(19) Europäisches Patentamt
European Patent Office
Office européen des brevets

(11) Publication number: **0 080 286 A1**

(12) # EUROPEAN PATENT APPLICATION

(21) Application number: 82305886.2

(22) Date of filing: 05.11.82

(51) Int. Cl.³: C 07 D 498/04, A 61 K 31/42
//
(C07D498/04, 263/00, 205/00),(A61K31/42, 31/43),(A61K31/42, 31/545)

(30) Priority: 25.11.81 GB 8135461
23.03.82 GB 8208409

(43) Date of publication of application: 01.06.83
Bulletin 83/22

(84) Designated Contracting States: BE CH DE FR GB IT LI NL SE

(71) Applicant: BEECHAM GROUP PLC, Beecham House Great West Road, Brentford Middlesex (GB)

(72) Inventor: Stirling, Irene, 38 Harrison Close, Reigate Surrey (GB)
Inventor: Bruton, Gordon, 61A Milton Road, Croydon Surrey (GB)

(74) Representative: Hesketh, Alan, Dr. et al, European Patent Attorney Beecham Pharmaceuticals Great Burgh Yew Tree Bottom Road, Epsom, Surrey, KT18 5XQ (GB)

(54) **Antibacterial compounds containing beta-lactams, processes for their preparation and their use.**

(57) A 3-carboxyclavam derivative with a butylidene moiety at the 2-position of the clavam nucleus, having the formula (III):

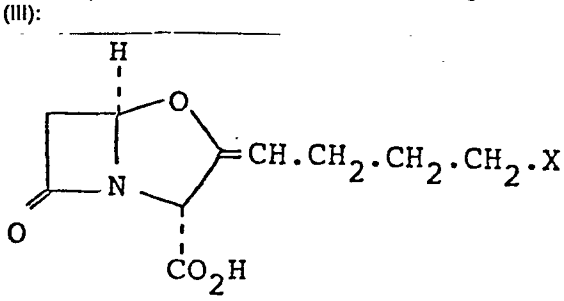

(III)

or a salt or ester thereof wherein X is hydrogen, hydroxy, substituted hydroxy, mercapto, substituted mercapto, azido, cyano, halo, nitro, amino, substituted amino, isothiocyanato, or X represents the residue of a carbon nucleophile or is an activated aryl or heteroaryl group,

processes of preparation and pharmaceutical composition comprising it either alone or in a synergistic composition with other beta-lactam antibacterial agents.

EP 0 080 286 A1

ANTIBACTERIAL AGENTS, PROCESSES FOR THEIR PREPARATION AND THEIR USE

This invention relates to novel β-lactam containing compounds, their preparation and their use, and in particular to a novel class of clavams. These compounds have antibacterial and β-lactamase inhibitory properties, and therefore are of use in the treatment of bacterial infection either alone or in a synergistic composition with other β-lactam antibacterial agents, such as penicillins and cephalosporins.

Clavulanic acid has the structure:

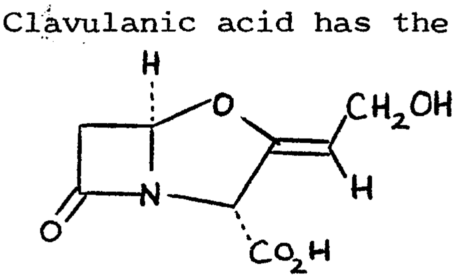

and is described in U.K. Patent Number 1508977. Many derivatives of that compound are known.

Clavulanic acid and its derivatives have in common the 3-carboxy-2-ethylidene clavam nucleus, i.e. the moiety

of the structure:

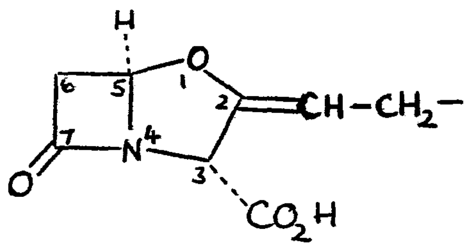

We have now produced a novel nucleus having a chain of four carbon atoms attached to the 2-position of the clavam.

Accordingly the present invention provides a 3-carboxyclavam derivative characterised in that it comprises a butylidene moiety at the 2-position of the clavam nucleus.

The invention therefore comprises compounds having the residue of the formula (I):

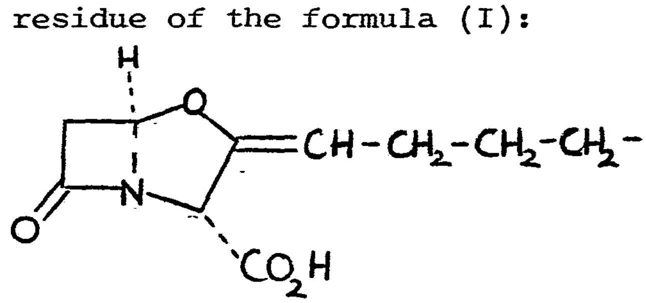

(I)

and salts and esters thereof.

The compounds of this invention can be considered to comprise 2-(4-hydroxybutylidene)clavam-3-carboxylic acid of the formula (II) and derivatives thereof:

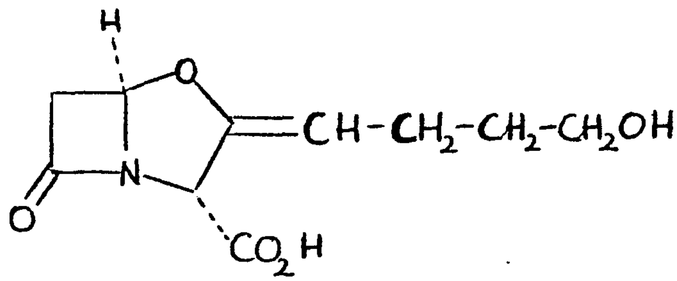

(II)

In particular the present invention includes compounds of the formula (III):

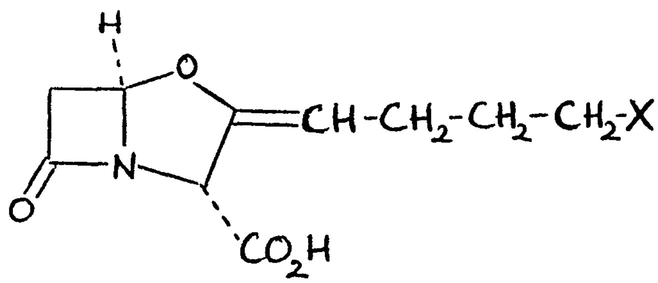

(III)

and salts and esters thereof, wherein X is hydrogen, hydroxy, substituted hydroxy such as etherified hydroxy or acylated hydroxy; mercapto, substituted mercapto such as etherified mercapto or acylated mercapto ; azido, cyano, halo, nitro, amino, isothiocyanato, substituted amino such as alkylated amino or acylated amino, or X represents the residue of a carbon nucleophile or is an activated aryl or heteroaryl group.

In one aspect X is hydroxy or substituted hydroxy such as etherified hydroxy or acylated hydroxy, mercapto or substituted mercapto such as etherified mercapto or acylated mercapto, azido, cyano, nitro, isothiocyanato, halo, amino or substituted amino such as alkylated amino or acylated amino, or X represents the

residue of a carbon nucleophile or is an activated aryl or heteroaryl group.

Suitably the compounds of the formula (III) are presented with Z stereochemistry about the double bond, that is to say they may be represented as in formula (IV):

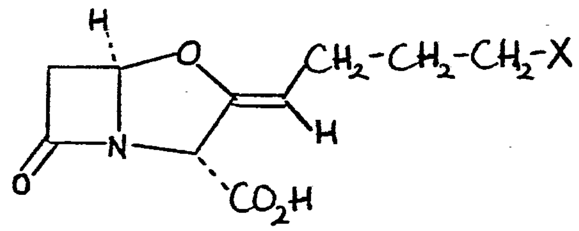

(IV)

Suitably X is hydroxy or substituted hydroxy. Examples of substituted hydroxy groups include $-OR^1$, $-OCOR^1$, $-OCSR^1$, $-OCO_2R^1$, $-OCS_2R^1$, $OCOSR^1$, $-OCSOR^1$, $-OSO_3H$, $-OPO_3H$, $-OSO_2R^1$ and $-OCONR^2R^3$ wherein $R^1$, $R^2$ and $R^3$ are independently substituted or unsubstituted hydrocarbon groups of 1 to 20 carbon atoms.

More suitably $R^1$ is $C_{1-10}$ alkyl, $C_{2-10}$ alkenyl, aryl $(C_{1-6})$ alkyl, heterocyclyl $(C_{1-6})$ alkyl, aryl $(C_{2-6})$ alkenyl, heteroaryl $(C_{1-6})$ alkyl, $C_{3-8}$ cycloalkyl, aryl, heteroaryl, heterocyclyl or $C_{3-8}$ cycloalkyl $(C_{1-6})$ alkyl, any of such groups being optionally substituted.

Suitable optional substituents for the group $R^1$ include $C_{1-6}$ alkyl, amino, $C_{1-6}$ alkanoylamino, mono-, di- and tri- $(C_{1-6})$ alkylamino, hydroxy, $C_{1-6}$ alkoxy, mercapto, $C_{1-6}$ alkylthio, heteroarylthio, arylthio, oxo, sulphamoyl, carbamoyl, amidino, guanidino, nitro, chloro, bromo, fluoro, carboxy and salts and esters thereof, $C_{1-6}$ alkanoyloxy, arylcarbonyl and heteroarylcarbonyl.

In one favourable aspect X is $C_{1-6}$ alkoxy or benzyloxy optionally substituted by one, two or three halogen atoms, cyano, azido, hydroxy, $C_{1-6}$ alkoxy, $C_{2-6}$ alkanoyloxy, $C_{1-4}$ alkylthio, aryloxy such as phenoxy, amino, $C_{1-6}$ alkanoylamino, carboxy or esterified carboxy.

Preferably X is methoxy or ethoxy.

In another aspect X is $C_{1-6}$ alkanoyloxy such as acetoxy, $C_{1-6}$ alkyl sulphonyloxy such as methanesulphonyloxy or arylsulphonyloxy such as p-toluenesulphonyloxy.

Suitably X is mercapto or substituted mercapto. Examples of substituted mercapto groups include $-SCOR^4$, $-S-R^4$, $-SCSR^4$, $-SCO_2R^4$, $-SCS_2R^4$, $-SCOSR^4$, $-SCSOR^4$, $-SSO_2R^4$, $-SCONR^5R^6$, $-S(O)R^4$, $-S(O)_2R^4$ and $-S-SR^4$, wherein $R^4$, $R^5$ and $R^6$ are independently substituted or unsubstituted hydrocarbon groups of 1 to 20 carbon atoms. Suitably substituents for $R^4$, $R^5$ and $R^6$ include those described hereinabove as being suitable as substitutents for $R^1$.

More suitably $R^4$ is a $C_{1-10}$ alkyl, $C_{2-10}$ alkenyl, aryl ($C_{1-6}$) alkyl, heterocyclyl ($C_{1-6}$) alkyl, aryl ($C_{2-6}$) alkenyl, $C_{3-8}$ cycloalkyl ($C_{1-6}$) alkyl, heterocyclyl, heterocyclyl ($C_{1-6}$) alkyl, aryl, heteroaryl, heteroaryl ($C_{1-6}$) alkyl or $C_{3-8}$ cycloalkyl group, any of such groups being optionally substituted.

Most suitably X is a group $-S(O)_nR^7$ wherein n is zero, one or two, and $R^7$ is benzyl, $C_{1-6}$ alkyl, $C_{2-6}$ alkenyl, aryl or heteroaryl, any of such groups $R^7$ being optionally substituted by one, two or three halogen atoms, azido, hydroxy, $C_{1-6}$ alkoxy, $C_{2-6}$ alkanoyloxy, $C_{1-4}$ alkylthio, aryloxy such as phenoxy, amino, $C_{1-6}$ alkanoylamino, carboxy or esterified carboxy.

Preferably X is $S(O)_nR^7$ wherein n is zero or two, and $R^7$ is $C_{1-6}$ alkyl such as methyl, benzyl, phenyl or tetrazolyl.

Suitably X is amino ($-NH_2$) or substituted amino. Examples of substituted amino groups include $-NHR^{10}$, $-NR^{10}R^{11}$, $-NR^{10}R^{11}R^{12}$, $-NHCX^1R^{10}$, $-NHCX^1ZR^{10}$, $-NHSO_2R^{10}$, $-NHCX^1NR^{10}R^{11}$, $-N(CX^1R^{12})R^{13}$, $-N(CX^1R^{12})CZR^{13}$, $-N(CX^1R^{12})ZR^{13}$, $-N(CX^1ZR^{12})R^{13}$ and $-N(CX^1ZR^{12})CX^1ZR^{13}$ wherein $X^1$ and Z are each oxygen or sulphur and $R^{10}$, $R^{11}$, $R^{12}$ and $R^{13}$ are independently substituted or unsubstituted hydrocarbon groups of 1 to 20 carbon atoms. Suitably substituents for $R^{10}$ - $R^{13}$ include those described hereinabove as being suitable as substituents for $R^1$. Additionally the groups $R^{10}$ and $R^{11}$, or alternatively the groups $R^{12}$ and $R^{13}$, may be joined to form a heterocyclic ring cohtaining from three to ten carbon atoms and not more than four heteroatoms selected from oxygen, nitrogen and sulphur.

More suitably $R^{10}$, $R^{11}$, $R^{12}$ and $R^{13}$ are independently selected from $C_{1-10}$ alkyl, $C_{2-10}$ alkenyl, aryl ($C_{1-6}$) alkyl, heterocyclyl ($C_{1-6}$) alkyl, aryl ($C_{2-6}$) alkenyl, $C_{3-8}$ cycloalkyl, $C_{3-8}$ cycloalkyl ($C_{1-6}$) alkyl, heterocyclyl, heterocyclyl ($C_{1-6}$) alkyl, aryl, heteroaryl and heteroaryl ($C_{1-6}$) alkyl, any of such groups being optionally substituted.

Most suitably X is amino, $C_{1-6}$ alkylamino such as methylamino, ethylamino, propylamino and butylamino, di-$(C_{1-6})$ alkylamino such as diethylamino, benzylamino, $C_{1-6}$ alkanoylamino such as acetamido, succinimido or phthalimide, any of such groups (except amino) being optionally substituted by one, two or three halogen atoms, azido, hydroxy, $C_{1-6}$ alkoxy, $C_{2-6}$ alkanoyloxy,= $C_{1-4}$ alkylthio, aryloxy such as phenoxy, amino, $C_{1-6}$ alkanoylamino, carboxy or esterified carboxy.

Preferably X is amino, $C_{1-6}$ alkylamino, benzylamino, $C_{1-6}$ alkanoylamino or di-$(C_{1-6})$ alkylamino.

Suitably X is an aryl or heteroaryl group for example bonded to the adjacent $-CH_2-$ group via a carbon atom, for example phenyl, furyl, pyrrolyl, thienyl and such ring systems fused to a benzene ring, any of such groups being optionally substituted.

Other heterocyclic groups for X include tetrazolyl and trioxoimidazolidinyl, optionally substituted with $C_{1-6}$ alkyl or $C_{1-6}$ alkoxycarbonyl.

Suitably X is the residue of a carbon nucleophile such as of the sub-formula $-CR^{14}R^{15}R^{16}$ wherein $R^{14}$ and $R^{15}$ are the same or different and at least one of them represents a group capable of producing a stabilised carbanion, such as an electron withdrawing group, and $R^{16}$ is hydrogen or a substituted or unsubstituted hydrocarbon group of 1 to 20 carbon atoms. Thus $R^{14}$ and $R^{15}$ may be selected from groups $-CX^{1}R^{10}$ and $CX^{1}ZR^{10}$ as previously described. Similarly $R^{16}$ may be defined as for $R^{10}$ hereinabove.

More suitably X is $-CH(COOR^{17})COR^{18}$ or $-CH(COR^{17})COR^{18}$ wherein $R^{17}$ and $R^{18}$ are independently selected from $C_{1-10}$ alkyl, $C_{3-8}$ cycloalkyl, aryl such as phenyl, aryl $(C_{1-6})$ alkyl such as benzyl, any of groups $R^{17}$ and $R^{18}$ being optionally substituted by one, two or three halogen atoms, azido, hydroxy, $C_{1-6}$ alkoxy, $C_{2-6}$ alkanoyloxy, $C_{1-4}$ alkylthio, aryloxy such as phenoxy, amino $C_{1-6}$ alkanoylamino, carboxy or esterified carboxy.

Preferably X is $-CH(COCH_3)_2$.

Suitably also X is azido or cyano. Aptly X is a hydrogen atom or a halo moiety for example iodo, bromo or chloro. Compounds wherein X is azido or halo are of particular interest as useful chemical intermediates.

The major utility of the compounds of the formulae (I) - (IV) and salts and esters thereof is as pharmaceuticals and accordingly the salts and esters of the compounds of the formulae (I) - (IV) are preferably pharmaceutically acceptable. The compounds of this invention both pharmaceutically acceptable and non-pharmaceutically acceptable may be used as intermediates and also as antibacterial agents or β-lactamase inhibitors in non-pharmaceutical usage such as a disinfectant or paint additive.

Suitable pharmaceutically acceptable salts of the compounds of the formula (I)-(IV) include metal salts such as aluminium, alkali metal salts such as sodium and potassium, and alkaline earth metal salts such as calcium or magnesium; and ammonium and substituted ammonium salts, for example those with lower alkylamines such as triethylamine, cycloalkylamines such as bicyclohexylamine, 1-ephenamine, N-ethylpiperidine and N-benzyl-β-phenethylamine.

Non-pharmaceutically acceptable salts of use as intermediates include the lithium and silver salts.

Suitable esters of the compounds of the formulae (I)-(IV) include those cleavable by biological methods such as enzy matic hydrolysis, in-vivo hydrolysis, and those cleavable by chemical methods such as hydrogenolysis, hydrolysis, electrolysis or photolysis.

Suitably the carboxylic acid is esterified by a group of the sub-formula (a), (b), (c), (d), (e) or (f):

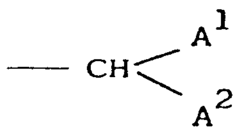
(a)

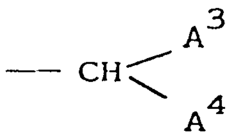
(b)

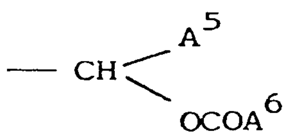
(c)

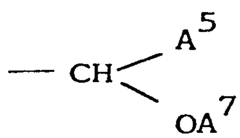
(d)

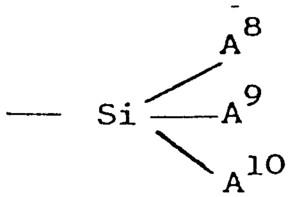
(e)

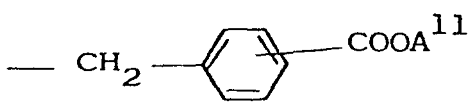
(f)

wherein $A^1$ is a hydrogen atom, $C_{1-6}$ alkanoyl or an $C_{1-5}$ alkyl group optionally substituted by $C_{1-7}$ alkoxy or $C_{1-7}$ carboxylic acyloxy, or an alkenyl or alkynyl group of up to 5 carbon atoms; $A^2$ is a hydrogen atom or a methyl group; $A^3$ is a phenyl group or a phenyl group substituted by a fluorine, chlorine or bromine atom or a nitro, $C_{1-3}$ alkyl or $C_{1-3}$ alkoxy group; $A^4$ is a hydrogen atom or a phenyl group or phenyl group substituted by a fluorine, chlorine or bromine atom or a nitro, $C_{1-3}$ alkyl or $C_{1-3}$ alkoxy group; $A^5$ is a hydrogen atom or a methyl group; $A^6$ is a $C_{1-4}$ alkyl, phenyl or $C_{1-4}$ alkoxy group or $A^5$ is

joined to $A^6$ to form a phthalidyl, dimethylphthalidyl or dimethoxyphthalidyl group; $A^7$ is a $C_{1-4}$ alkyl, phenyl, chlorophenyl or nitrophenyl group; $A^8$ is a $C_{1-4}$ alkyl or phenyl group; $A^9$ is a $C_{1-4}$ alkyl or phenyl group; $A^{10}$ is $C_{1-4}$ alkyl; and $A^{11}$ is $C_{1-4}$ alkyl: or $CHA^1A^2$ is a phenacyl or bromophenacyl group.

Favourably $A^1$ is a hydrogen atom or a methyl, ethyl, vinyl or ethenyl group. Favourably $A^2$ is a hydrogen atom. Favourably $A^3$ is a phenyl, p-bromophenyl, p-methoxyphenyl or p-nitrophenyl group. Favourably $A^4$ is a hydrogen atom. Favourably $A^6$ is a methyl, t-butyl or ethoxy group or is joined to $A^5$. Favourably $A^7$ is a methyl group.

Preferred groups of the sub-formula (a) include the methyl, ethyl and acetonyl groups.

Preferred groups of the sub-formula (b) include the benzyl and p-nitrobenzyl groups.

Preferred groups of the sub-formula (c) include the acetoxymethyl, pivaloyloxymethyl, α-ethoxycarbonyloxymethyl and phthalidyl groups.

A preferred group of the sub-formula (d) is the methoxymethyl group.

Preferred groups of the sub-formula (e) include the trimethylsilyl, tert-butyldimethylsilyl and tert-butyldiphenylsilyl groups.

A preferred group of the sub-formula (f) is p-methoxycarbonylbenzyl.

Particularly preferred esterifying groups are the p-nitrobenzyl and phthalidyl groups.

Pharmaceutically acceptable *in-vivo* hydrolysable esters are those esters which hydrolyse in the human body to produce the parent acid or its salt. Such esters may be identified by administration to a test animal such as a rat or mouse by intravenous administration and thereafter examining the test animal's body fluids for the presence of the compound of the formulae (I)-(IV) or salt.

Suitable esters of this type include those of sub-formula (c) as hereinbefore defined.

Further suitable esters include di($C_{1-6}$) alkylamino $C_{1-6}$ alkyl esters such as dimethylaminomethyl, dimethylaminoethyl, diethylaminomethyl and diethylaminoethyl.

Esters of the compounds of the formulae (I)-(IV) such as those of the sub-formula (a)-(f) if desired, may be presented in the form of their acid addition salts if an amino group is present in group X. The acid used to form the salt will most suitably be pharmaceutically acceptable, but non-pharmaceutically acceptable acid addition salts are also envisaged, for example as intermediates in the preparation of the pharmaceutically acceptable salts by ion exchange. Suitable pharmaceutically acceptable acid addition salts include those of inorganic and organic acids, such as hydrochloric, phosphoric, sulphuric, methanesulphonic, toluenesulphonic, citric, malic, acetic, lactic, tartaric, propionic and succinic acid.

Most suitably the acid addition salt is provided as a solid and preferably as a crystalline solid.

Compounds of this invention when in crystalline form may be solvated, for example hydrated.

The present invention provides a pharmaceutical composition which comprises a compound of the formula (I) or a pharmaceutically acceptable salt or pharmaceutically acceptable ester thereof and a pharmaceutically acceptable carrier.

The compositions of the invention include those in a form adapted for oral, topical or parenteral use and may be used for the treatment of infection in animals for example mammals including humans.

Suitable forms of the compositions of this invention include tablets, capsules, creams, syrups, suspensions, solutions, reconstitutable powders and sterile forms suitable for injection or infusion. Such compositions may contain conventional pharmaceutically acceptable materials such as diluents, binders, colours, flavours, preservatives, disintegrant and the like in accordance with conventional pharmaceutical practice in the manner well understood by those skilled in the art of formulating antibiotics.

Injectable or infusable compositions of a compound of the invention are particularly suitable as high blood levels of the compound can occur after administration by injection or infusion. Thus, one preferred composition aspect of this invention comprises a compound of the invention in sterile form and most suitably in sterile crystalline form.

The injectable solution of the compound of this invention may be made up in a sterile pyrogen-free liquid such as water, aqueous ethanol or the like.

An alternative approach to administering the compounds of this invention is to utilise an injectable suspension. Such suspensions may be made up in sterile water; sterile saline or the like, and may also contain suspending agents such as polyvinylpyrrolidone, lecithin or the like. Alternatively such compositions may be prepared in an acceptable oil suspending agent such as arachis oil or its equivalent. For use in such suspensions the compounds of this invention should be in the form of fine particles.

Unit dose compositions comprising a compound of this invention adapted for oral administration form a further suitable composition aspect of this invention.

Unit dose compositions comprising a compound of this invention adapted for topical administration are also presented by this invention. In this instance 'topical administration' also includes local administration to internal surfaces of mammary glands of cattle, for example during the treatment of mastitis by intra-mammary administration.

The compound of the invention may be present in the composition as sole therapeutic agent or it may be present together with other therapeutic agents such as a penicillin or cephalosporin. Considerable advantages accrue from the

inclusion of a penicillin or cephalosporin which shows instability to β-lactamases since the resulting composition shows enhanced effectiveness (synergy). Suitable penicillins, cephalosporins or other β-lactam antibiotics for inclusion in such synergistic compositions include not only those known to be highly susceptible to β-lactamases but also those which have a degree of intrinsic resistance to β-lactamases.

Suitable penicillins for inclusion in the compositions of this invention include benzylpenicillin, phenoxymethylpenicillin, carbenicillin, azidocillin, propicillin, ampicillin, amoxycillin, epicillin, ticarcillin, cyclacillin, pirbenicillin, azlocillin, mezlocillin, sulbenicillin, piperacillin, and other known penicillins including pro-drugs therefor such as their in vivo hydrolysable esters such as the acetoxymethyl, pivaloyloxymethyl, α-ethoxycarbonyloxyethyl or phthalidyl esters of ampicillin, benzylpenicillin or amoxycillin, and aldehyde or ketone adducts of penicillins containing a 6-α-aminoacetamide side chain (such as hetacillin, metampicillin and analogous derivatives of amoxycillin) or α-esters of carbenicillin or ticarcillin such as their phenyl or indanyl α-esters.

A further group of suitable penicillins of interest for inclusion in the compositions of this invention include benzylpenicillin, phenoxymethylpenicillin, carbenicillin, azidocillin, propicillin, amoxycillin, epicillin, ticarcillin, cyclacillin, pirbenicillin, azlocillin, mezlocillin, sulbenicillin, piperacillin, and other known penicillins including pro-drugs therefor such as their <u>in vivo</u> hydrolysable esters such as the acetoxymethyl, pivaloyloxymethyl, α-ethoxycarbonyloxyethyl or phthalidyl esters of benzylpenicillin or amoxycillin, and α-esters of carbenicillin or ticarcillin such as their phenyl or indanyl α-esters.

Suitable cephalosporins for inclusion in the compositions of this invention include cefatrizine, cephaloridine, cephalothin, cefazolin, cephalexin, cephacetrile, cephamandole nafate, cephapirin, cephradine, 4-hydroxycephalexin, cefaparole, cephaloglycin, cefoperazone and other known cephalosporins or pro-drugs thereof.

Such compounds are frequently used in the form of a salt or hydrate or the like.

Naturally if the penicillin or cephalosporin present in the composition is not suitable for oral administration then the composition will be adapted for parenteral administration.

Highly favoured penicillins for use in the compositions of this invention include ampicillin, amoxycillin, carbenicillin and ticarcillin. Such penicillins may be used as a pharmaceutically acceptable salt such as the sodium salt. Alternatively the ampicillin or amoxycillin may be used in the form of fine particles of the zwitterionic form (generally as ampicillin trihydrate or amoxycillin trihydrate) for use in an injectable suspension, for example in the manner hereinbefore described for a compound of this invention.

The preferred penicillin for use in the synergistic composition is amoxycillin, for example as its sodium salt or trihydrate.

Particularly suitable cephalosporins for use in the compositions of this invention include cephaloridine and cefazolin which may be in the form of a pharmaceutically acceptable salt for example the sodium salt.

When present together with a cephalosporin or penicillin, the ratio of a compound of the invention to the penicillin or cephalosporin agent may vary over a wide range of ratios, such as from 10:1 to 1:10 for example about 3:1, 2:1, 1:1, 1:2, 1:3, 1:4, 1:5 or 1:6, (wt/wt, based on pure free antibiotic equivalent).

Orally administrable compositions containing a compound of the invention will normally contain relatively more synergist than corresponding injectable compositions.

The total quantity of a compound of the invention in any unit dosage form will normally be between 25 and 1000 mg and will usually be between 50 and 500 mg, for example about 62.5, 100, 125, 150, 200 or 250 mg.

Compositions of this invention may be used for the treatment of infections of animals, for example mammals including humans, such infections being *inter alia* of the respiratory tract, the urinary tract and soft tissues in humans and mastitis in cattle.

Normally between 50 and 3000 mg of the compounds of the invention will be administered per day, for example at 1-6 doses, more usually as 2, 3 or 4 doses. However, for the treatment of more severe systemic infections or infections of particularly intransigent organisms higher doses may be used in accordance with clinical practice.

The penicillin or cephalosporin in the synergistic composition of this invention will normally be present at

approximately the amount at which it is conventionally used which will usually be expected to be from about 62.5 to 3000 mg per dose, more usually about 125, 250, 500 or 1000 mg per dose.

One particularly favoured composition of this invention will contain from 150 to 1000 mg of amoxycillin as the trihydrate or sodium salt and from 25 to 500 mg of a compound of this invention.

A further particularly favoured composition of this invention will contain from 150 to 1000 mg of ampicillin or a pro-drug therefor and from 25 to 500 mg of a compound of this invention.

Most suitably this form of composition will contain ampicillin trihydrate, ampicillin anhydrate, sodium ampicillin, hetacillin, pivampicillin hydrochloride, bacampicillin hydrochloride, or talampicillin hydrochloride. Most suitably this form of the composition will contain a compound of this invention when in crystalline form.

Most suitably the preceding composition will contain from 200 to 700 mg of the penicillin component.

Most suitably the preceding composition will comprise from 50 to 250 mg of a compound of this invention preferably in crystalline form.

Such compositions may be adapted for oral or parenteral use except when containing an *in vivo* hydrolysable ester of ampicillin or amoxycillin in which case the compositions will not be adapted for parenteral administration.

Another particularly favoured composition of this invention will contain from 200 to 2000 mg of carbenicillin, ticarcillin or a pro-drug therefor and from 50 to 500 mg of a compound of the invention.

Suitably this form of composition will contain di-sodium carbenicillin. Suitably this form of the composition will contain di-sodium ticarcillin.

More suitably this form of the composition will contain from 75 to 250 mg of a compound of this invention preferably in crystalline form. Such compositions containing di-salts of carbenicillin and ticarcillin will be adapted for parenteral administration.

The present invention also provides a method of treating bacterial infection in humans or domestic mammals which comprises the administration of a composition of this invention.

Commonly the infection treated will be due to a strain of Staphylococcus aureus, Klebsiella aerogenes, Escherichia coli, Proteus sp., Bacteroides fragilis or the like. The organisms believed to be most readily treated by an antibacterially effective amount of a compound of this invention is Staphylococcus aureus. The other organisms named are more readily treated by using a synergistically effective amount of the compound of the invention and a penicillin or cephalosporin. The administration of the two components may take place separately but in general we prefer to use a composition containing both the synergist and the penicillin or cephalosporin.

The indications for treatment include respiratory tract and urinary tract infections in humans and mastitis in cattle.

The present invention also provides a process for the preparation of a compound of the formula (III) and salts and esters thereof which process comprises the reduction of a compound of the formula (V):

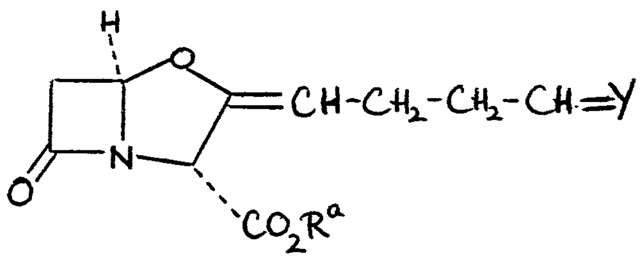

(V)

wherein $R^a$ is hydrogen or a carboxy-blocking group and Y is an oxygen or sulphur atom, and thereafter if necessary:

i) converting a compound of the formula (III) wherein X is hydroxy or mercapto to a compound of the formula (III) wherein X is not hydroxy or mercapto,

ii) removing any carboxy-blocking group $R^a$,

iii) converting the product into a salt or ester.

Preferably in the compounds of the formula (V) Y is an oxygen atom. The compounds of the formula (V) are novel and are of use as intermediates. In addition they are active antibacterial agents in their own right.

The compounds of the formula (V) wherein Y is sulphur when used as intermediates are not normally isolated but are prepared and reacted <u>in situ</u>.

Compounds of the formula (V) may be converted to compounds of the formula (III) wherein X is hydroxy or mercapto by reduction in conventional manner. A suitable method of reduction utilises a complex hydride such as a borohydride, for example lithium borohydride, sodium or potassium borohydride, sodium cyanoborohydride, di-isobutyl aluminium hydride; or a reagent such as aluminium tri-isopropoxide. Such reaction may be performed in an inert organic solvent such as a hydrocarbon, a chlorinated hydrocarbon or an ether, for example toluene, cyclohexane, diethyl ether, heptane, hexane, dichloromethane or tetrahydrofuran. The reaction may be performed at any non-extreme temperature for example -30°C to +60°C, more suitably 0°C to +30°C and most conveniently at ambient temperature.

Compounds of the formula (III) wherein X is hydroxy or mercapto may be converted to the compounds of the formula (III) where X is not OH or SH in conventional manner, using methods appropriate to the formation of each functional group.

For example, compounds wherein X is halo may be prepared by reaction with a halogenating agent such as a non-metallic halide for example phosphorus trichloride, phosphorus tribromide, thionyl chloride, thionyl bromide or the halogenating agent may be a hydrocarbonsulphonyl halide generally used in the presence of halide ions, for example an alkanesulphonyl halide such as methanesulphonylchloride or an arylsulphonyl halide such as p-toluenesulphonyl chloride; the source of halide ions may be lithium halide or an amine salt. Preferably in the halogenation reaction a base is present such as pyridine, and the reaction is preferably performed at a depressed or ambient temperature for example -60°C to +20°C. The compounds of the formula (III) wherein X is iodo are most conveniently prepared by a halogen exchange reaction, reacting the corresponding chloro or bromo compound with a source of iodide ions such as lithium iodide.

Compounds of the formula (III) wherein X is substituted hydroxy or substituted mercapto may be prepared by conventional methods, for example by etherification or acylation. Etherification of -OH or -SH may be effected by the reaction of a diazoalkane

in the presence of a Lewis acid catalyst for example boron trifluoride preferably at a depressed or ambient temperature in an inert solvent. Acylation of -OH or -SH may be effected by reaction with an acylating agent eg. RCY-T or $RSO_2$-T wherein T is a leaving group displaceable by a nucleophile. Suitably T is halo such as chloro or bromo, or a sulphonyloxy moiety, for example $C_{1-6}$ alkyl- or aryl- sulphonyloxy such as a mesylate or tosylate, or T is a carboxylate moiety, for example a $C_{1-6}$ alkanoyloxy group optionally substituted by 1 to 3 halogen atoms, in particular acetoxy or is an arylcarbonyloxy for example benzoyloxy. Preferably such reaction is performed in the presence of a base such as an alkali or alkaline earth metal salt, in particular lithium, sodium or potassium carbonates; or an organic base such as an amine for example pyridine or a tertiaryamine and in particular triethylamine. Suitable solvents include chloroform, dichloromethane, tetrahydrofuran, dimethylformamide, dimethylsulphoxide and acetone. Generally the acylation reaction is performed at a non-extreme temperature for example at an ambient or slightly depressed temperature, such as -20°C to +20°C. Alternatively T is a hydroxy group or hydrocarbyloxy for example $C_{1-6}$ alkoxy or aryloxy such as phenoxy. When T is hydroxy preferably Y is oxygen and a dehydrating agent such as dicyclohexylcarbodi-imide is present.

Compounds of the formula (III) wherein X is azido may be prepared by the reaction of an azide with a compound of the formula (III) wherein X is a readily displaceable group such as a halogen atom (chlorine or bromine) or a sulphonyloxy group. The

azide is suitably an alkali metal azide such as sodium azide or an organic azide such as tetramethylguanidinium azide or tetrabutylammonium azide. The reaction is preferably performed in an inert polar organic solvent such as acetonitrile, dimethylsulphoxide, ethyl acetate, dichloromethane, acetone or tetrahydrofuran at a non-extreme temperature.

The compounds of the formula (III) wherein X is amino may be prepared by the reduction of the aforementioned azido group, for example via catalytic hydrogenation such as over Palladium on carbon, or via dissolving metal reduction for example using zinc and aqueous hydrochloric acid.

Compounds of the formula (III) wherein X is substituted amino may be prepared by the reaction of a compound of the formula (III) wherein X is amino, for example by alkylation and/or acylation.

Alkylation of the primary amine ($-NH_2$) may be effected by reaction with a compound RX" wherein X" is a leaving group to form a secondary amine (-NHR). This secondary amine then may be reacted with another compound RX" (which may be the same or different as the first) to a form a tertiary amine ($-NR_2$), and subsequently, if desired, a quaternary amine ($-NR_3^{\oplus}$) may be prepared,

wherein the substituent groups are the same or different. Suitable groups X" include halo such as iodo and bromo, or a sulphonate such as a $C_{1-6}$ alkyl- or aryl- sulphonate for example methanesulphonate or p-toluenesulphonate. Such alkylation reactions are conveniently performed in an inert organic solvent such as dimethylformamide, acetonitrile or dichloromethane, preferably in the presence of strong non-nucleophilic organic base at a non-extreme temperature such as $-20^{o}C$ to $+50^{o}C$, preferably $-10^{o}C$ to $+20^{o}C$ and most conveniently between $0^{o}C$ and ambient. Suitable organic bases include 1,5-diazabicyclo[4.3.0]non-5-ene and 1,8-diazabicyclo[5.4.0]undecene.

Primary, secondary and tertiary amines also may be prepared by the reaction of a compound of the formula (III) wherein X is a good leaving group, such as tosyloxy with a secondary amine ($RNHR^{o}$) to form a tertiary amine. One or both of the groups R and $R^{o}$ may then be removed, for example by hydrogenation to provide a secondary or primary amine.

Acylation of the primary amine ($-NH_2$) may be effected by reaction with an acylating agent. Apt acylating agents include any N-acylating compound suitable for the performance of analogous reactions with 6-aminopenicillanic acid or 7-aminocephalosporanic acid or salts or esters thereof, for example an acid halide, an anhydride or mixed anhydride or other reactive derivative, such as that produced by the reaction of an acid with an enzyme or a condensation-promoting agent such as dicyclohexylcarbodi-imide. Thus in general the acylating agent is of the formula RX" where X" is a displaceable group, for example halo, sulphonate, carboxylate. The acylating agent also may be a ketene or isocyanate or isothiocyanate or an activated imide. Preferably the acylation reaction is performed in the presence of a base, for example an inorganic base such as an alkali or alkaline earth

metal salt, in particular lithium, sodium or potassium carbonates; or an organic base such as an amine for example triethylamine. Suitably the reaction is performed in an aprotic organic solvent such as dimethylformamide, acetonitrile, ethyl acetate, dimethyl sulphoxide; water may be added to aid solubility if necessary. The reaction is suitably performed at a non-extreme temperature, for example between $-20^{o}C$ and $+40^{o}C$ and preferably at ambient temperature.

Acylation may be effected on the primary amine ($-NH_2$) once or twice to form the mono-acyl or di-acyl derivatives, in the di-acyl case the acyl groups may be the same or different. Alternatively acylation may be effected on a secondary amine (-NHR) to form the N-alkyl-N-acyl derivative.

Di-acyl derivatives wherein the two acyl groups are joined so as to form a heterocyclylic ring are most conveniently prepared by the reaction of a compound of the formula (III) wherein X is OH with a compound of the formula : $HNR_AR_B$ wherein $R_A$ and $R_B$ from the heterocyclylic ring system in the presence of an azodicarboxylate compound of the formula (VI):

$$R^{19}O.CO\text{-}N{=}N\text{-}CO.OR^{20} \qquad (VI)$$

wherein $R^{19}$ and $R^{20}$ are independently $C_{1-6}$ alkyl, aryl $C_{1-6}$ alkyl or aryl; and a compound of the formula **(VII):**

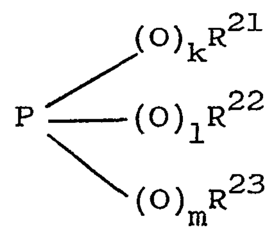

(VII)

wherein k, l and m are independently zero or one, and $R^{21}$, $R^{22}$ and $R^{23}$ are independently $C_{1-6}$ alkyl, aryl $C_{1-6}$ alkyl or aryl. The reaction is suitably performed in an inert organic solvent such as tetrahydrofuran and benzene, at a non-extreme temperature such as $-20^{o}C$ to $+100^{o}C$, usually at $-50^{o}C$ to $50^{o}C$ and conveniently at ambient temperature.

The foregoing reaction may also be used to prepare di-acyl derivatives wherein the two acyl groups are not joined. If one or both of the two acyl groups are removable for example hydrogenolysable then a mono-acyl derivative or primary amine ($-NH_2$) may be formed.

Compounds of the formula (III) wherein X is hydrogen may be prepared from the corresponding compounds wherein X is halo, for example iodo, by reduction. Mention may be made of tri-n-butyl tin hydride, Zinc in acetic acid, sodium borohydride and sodium cyanoborohydride.

Compounds of the formula (III) wherein X is cyano may be prepared by the reaction of a compound of

the formula (III), wherein X is OH with hydrogen cyanide in the presence of a compound of the formula (VI) and a compound of the formula (VII) under similar reaction conditions to those described above.

Compounds of the formula (III) wherein X is an activated aryl or heteroaryl group may be prepared by the reaction of a compound of the formula (III) wherein X is OH with an appropriate aromatic or heteroaromatic compound. By "activated aryl or heteroaryl" we mean a group derived from an aromatic or heteroaromatic compound that is sufficiently activated to undergo a Friedel-Crafts alkylation. Suitably such compounds include phenol, phenol substituted by one, two or three atoms or groups selected from hydroxy, halo, $C_{1-6}$ alkoxy, $C_{1-6}$ alkyl or $C_{3-8}$ cycloalkyl; furyl optionally substituted by $C_{1-6}$ alkyl, thienyl optionally substituted by $C_{1-6}$ alkyl, pyrrolyl optionally substituted by $C_{1-6}$ alkyl, or any of the aforementioned ring systems fused to an optionally substituted benzene ring. A catalyst is employed which is one known to catalyst reactions known as Friedel-Crafts reactions. Suitable catalysts include aluminium trichloride, zinc chloride, zinc bromide, antimony pentachloride, ferric chloride, stannic chloride, boron trifluoride etherate and chemical equivalents thereof. Of these boron trifluoride etherate is preferred. The reaction is generally performed in the presence of an inert organic solvent, preferably a non-polar solvent such as a halogenated hydrocarbon for example

chloroform, carbon tetrachloride or dichloromethane. Suitably the reaction is performed at a depressed or moderate temperature, i.e. between +50°C and -70°C, preferably between 0°C and -30°C. Preferably the reaction is performed under an inert atmosphere.

Compounds of the formula (III) wherein X is the residue of a carbon nucleophile may be prepared from compounds of the formula (III) wherein X is a displaceable group by reaction with a carbon nucleophile. The displaceable group may be halo such as chloro, bromo or iodo or aryl- or $C_{1-6}$ alkyl- sulphonyloxy for example p-toluenesulphonyloxy or methanesulphonyloxy. Reaction is suitably performed in an aprotic organic solvent at a depressed or ambient temperature for example -70°C to +25°C, preferably at -20°C to 0°C. Suitable solvents include tetrahydrofuran, dimethylformamide or dimethylsulphoxide. The carbon nucleophile may be a compound R-H in which case a base is present, or the carbon nucleophile may be presented as a salt. The cation in such a salt is suitably a metal ion or an ammonium ion, for example sodium, potassium, thallium or a tetra-$C_{1-6}$- alkyl ammonium cation. Thallium salts are particularly preferred.

Suitable carboxyl-blocking derivatives for the group $-CO_2R^a$ in formulae (V) include salt, ester and anhydride derivatives of the carboxylic acid. The

derivative is one which may readily be cleaved at a later stage of the reaction.

Suitable ester-forming carboxyl-blocking groups are those which may be removed under conventional conditions, and include those of sub-formulae (a)-(f) as hereinbefore defined. Such groups for $R^a$ include benzyl, p-methoxybenzyl, 2,4,6-trimethylbenzyl, 3,5-di-t-butyl-4-hydroxybenzyl, benzoylmethyl, p-nitrobenzyl, 4-pyridylmethyl, 2,2,2-trichloroethyl, 2,2,2-tribromoethyl, acetonyl, 2-methylallyl, t-butyl, t-amyl, diphenylmethyl, triphenylmethyl, methyl, ethyl, allyl, adamantyl, o-nitrobenzyl, 2-benzyloxyphenyl, tetrahydrofur-2-yl, tetrahydropyran-2-yl, methoxymethyl, a silyl, stannyl or phosphorus-containing group, or an in-vivo hydrolysable ester.

Suitable salts include both pharmaceutically acceptable and non-pharmaceutically acceptable salts, for example inorganic salts such as metal salts (silver and mercuric), or alkali metal salts (sodium and lithium) and tertiary amine salts.

The carboxyl group may be regenerated from any of the above esters by usual methods appropriate to the particular $R^a$ group, for example, acid - and base - catalysed hydrolysis, or by enzymically - catalysed hydrolysis, photolysis or by hydrogenation. The hydrolysis must of course be carried out under conditions to which the groups on the rest of the molecule are stable.

The present invention further provides a process for the preparation of a compound of the formula (V) which process comprises the rearrangement of a compound of the formula (VIII):

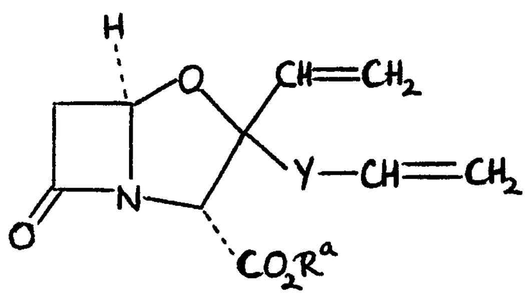

(VIII)

or a precursor thereof, wherein $R^a$ and Y are as defined in relation to formula (V):

Preferably $R^a$ is an esterifying group.

Preferably in the compounds of the formula (VIII) Y is an oxygen atom. It should be realised that the compounds of the formula (VIII) may be presented in either of the two epimeric forms at the ring carbon atom adjacent to the vinyl group, or may be presented as a mixture of epimeric forms. The compounds of the formula (VIII) are novel and as such form part of this invention.

The compounds of the formula (VIII) are not normally isolated but are formed and reacted in situ.

The rearrangement is generally carried out by heating a solution of the compound of the formula (VIII) in an inert high boiling solvent to a moderately high temperature, for example generally higher than about 70°C but less than that temperature at which any significant thermal decomposition occurs. Suitably the temperature is between 70°C and 150°C, more suitably 90°C - 130°C and preferably at about 110°C. Convenient solvents for this process include aromatic hydrocarbons such as benzene and mono, di- or tri- $C_{1-4}$ alkyl benzenes, for example toluene, xylene and cumene. Toluene is a particularly suitable solvent as it has a boiling-point of about 110°C and the reaction may be conveniently carried out in this solvent under reflux.

As has been mentioned the compounds of the formula (VIII) are normally prepared and isolated in situ, and it is not absolutely necessary for the rearrangement of formula (VIII) to formula (V) that high temperatures be used. However it is more convenient to do so as the formation of the compounds of the formula (VIII) is conducted at high temperatures.

The time for which the reaction is allowed to proceed depends on the particular starting-material selected, but a suitable time period may be readily

determined by monitoring the progress of the reaction by, for example, high pressure liquid chromatography or thin layer chromatography on silica using for example ethyl acetate: cyclohexane as a developing solvent.

The compounds of the formula (VIII) may be prepared by the elimination of the elements of H-Q from a compound of the formula (IX):

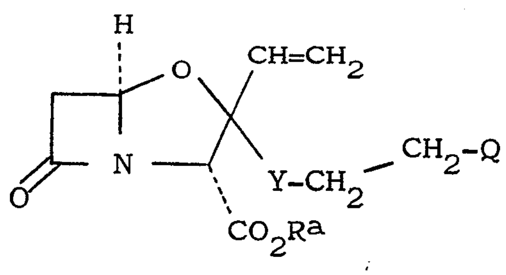

(IX)

wherein Y and $R^a$ are as hereinbefore defined and Q is a group that will undergo the β- el imination. For example Q may be a sulphoxide -S(O)R or a selenoxide -Se(O)R or a xanthate -OC(S)SR. The elimination is generally carried out at an elevated temperature such as between 70°C and 150°C, preferably at about 110°C, in an aromatic hydrocarbon. Suitable values for Q include -OC(S)SPh, -Se(O)Ph and -S(O)Ph and variants thereof wherein the phenyl ring is inertly substituted. Favourably Q is -Se(O)Ph or $Se(O)CH_3$.

The compounds of the formula (IX) may be converted in "one-pot" to the compound of the formula (V) without isolation of the compound of the formula (VIII).

The compounds of the formula (IX) may be prepared by replacement of halo, for example bromo, by the group Q in the compounds of the formula (X):

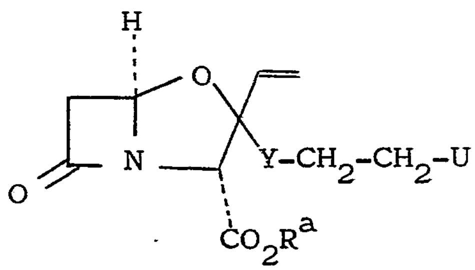

(X)

wherein $R^a$ and Y are as hereinbefore defined and U is a displaceable group, for example tosylate, mesylate or halo such as bromo or chloro.

For example Q is -Se(O)Ph and substituted variants thereof may be prepared by the oxidation of the corresponding selenide -Se-Ph, etc, with an oxidising agent such as sodium metaperiodate, potassium permanganate or m-chloroperbenzoic acid. The selenide may be prepared by the reaction of a compound of the formula (X) with an appropriate selenol in the presence of base, or selenide anion. Compounds wherein Q is -S(O)Ph and substituted variants thereof may be prepared analogously.

Compounds of the formula (X) may be prepared by the reaction of a compound of the formula (XI):

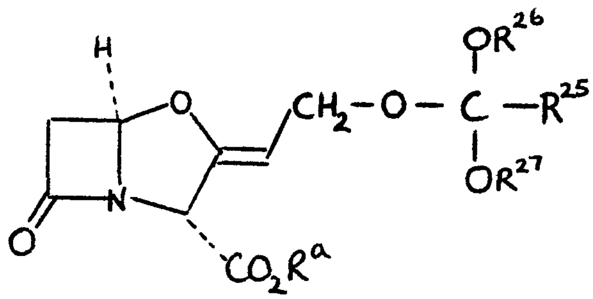

(XI)

wherein $R^a$ is as hereinbefore defined, $R^{25}$ is a hydrogen atom or a hydrocarbon group of 1 to 20 carbon atoms and $R^{26}$ and $R^{27}$ are independently $C_{1-6}$ alkyl groups; with a compound of the formula (XII):

$$U - CH_2 - CH_2 - YH \qquad \text{(XII)}$$

wherein U is halo, such as bromo or chloro, and Y is oxygen or sulphur. The reaction may conveniently be performed in a polar non-nucleophilic solvent such as dimethylformamide at a temperature of ambient to 60°C at a reduced pressure.

Compounds of the formula (XI) may be prepared by the reaction of a compound of the formula (XIII):

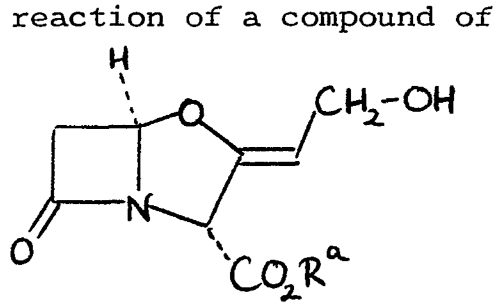

(XIII)

with an appropriate orthoester of the formula (XIV):

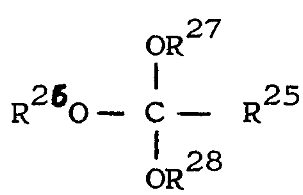

(XIV)

wherein $R^{25}$, $R^{26}$ and $R^{27}$ are as hereinbefore defined, and $OR^{28}$ is a leaving group, such as a $C_{1-6}$ alkoxy group for example methoxy. It is believed that this reaction is an equilibrium reaction and therefore it is preferred that $R^{28}O$ be chosen such that $R^{28}OH$ is removed or consumed during the reaction, such removal for example could be conveniently performed by evaporation, or under reduced pressure, if $R^{28}OH$ is volatile.

The compounds of the formula (X) may also be prepared by the reaction of a compound of the formula (XV):

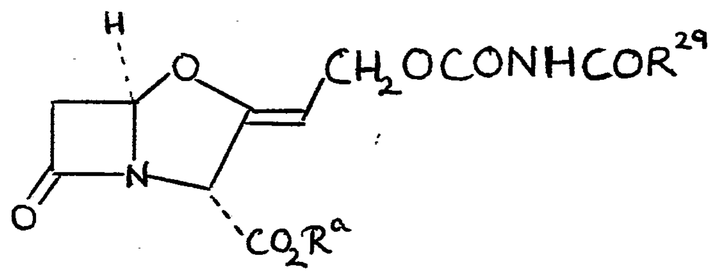

(XV)

wherein $R^a$ is a carboxy-blocking group and $R^{29}$ is an inert moiety such as alkyl, alkenyl, alkynyl, aryl, alkoxy, aryloxy, aralkoxy, aralkyl; with a compound of the formula (XII). Preferably this reaction is performed at ambient or an elevated temperature between 20°C and 80°C, such as 50°C, in an inert organic solvent, for example dimethylformamide.

The compounds of the formula (XV) are described in European Patent Application Publication Number 0002320.

The present invention also provides a process for the preparation of a compound of the formula (III) and salts and esters thereof which process comprises the reduction of a compound of the formula (XVI):

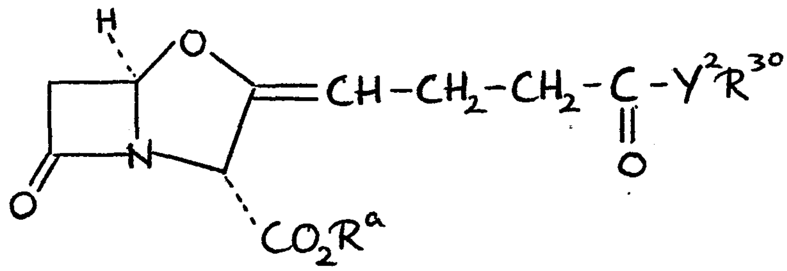

(XVI)

wherein $R^a$ is a hydrogen atom or carboxy-blocking group, $Y^2$ is oxygen or sulphur, and $R^{30}$ is an esterifying group; and thereafter if necessary:

i) converting a compound of the formula (III) wherein X is hydroxy to a compound of the formula (III) wherein X is not hydroxy,

ii) removing any carboxy-blocking group $R^a$,

iii) converting the product into a salt or ester.

Compounds of the formula (XVI) may be converted to compounds of the formula (III) wherein X is hydroxy by reduction in conventional manner. A suitable method of reduction utilises a complex hydride such as a borohydride, optionally sulphurated, for example lithium borohydride, sodium borohydride, potassium borohydride, sodium cyanoborohydride,

or di-isobutyl aluminium hydride. Such reaction may be performed in an inert organic solvent such as a hydrocarbon, a chlorinated hydrocarbon or an ether, for example toluene, cyclohexane, diethyl ether, heptane, hexane, dichloromethane or tetrahydrofuran. The reduction may be performed at any non-extreme temperature for example -30°C to +60°C, more suitably 0°C to +30°C and most conveniently at ambient temperature. Preferably $R^a$ is hydrogen in the above reaction.

Suitably $R^{30}$ is a substituted or unsubstituted hydrocarbon radical of 1 to 20 carbon atoms. More suitably $R^{30}$ is $C_{1-10}$ alkyl, $C_{2-10}$ alkenyl, $C_{2-10}$ alkynyl, aryl ($C_{1-6}$) alkyl, aryl ($C_{2-6}$) alkenyl, heteroaryl ($C_{1-6}$) alkyl, $C_{3-8}$ cycloalkyl, aryl, heteroaryl, $C_{3-8}$ cycloalkyl ($C_{1-6}$) alkyl, heterocyclyl or heterocyclyl ($C_{1-6}$) alkyl, any of such groups being optionally substituted.

Preferably $R^{30}$ is methyl, ethyl or benzyl.

Suitably $Y^2$ is oxygen.

The compounds of the formula (XVI) are novel and are of use as chemical intermediates. Furthermore they are of use as antibacterial agents, in their own right, or as β-lactamase inhibitors. In addition the compounds of the formula (XVI) wherein $R^{30}$ is replaced by a pharmaceutically acceptable salt-forming cation such as sodium or potassium, are active and useful antibacterial agents.

The present invention also provides a process for the preparation of a compound of the formula (XVI):

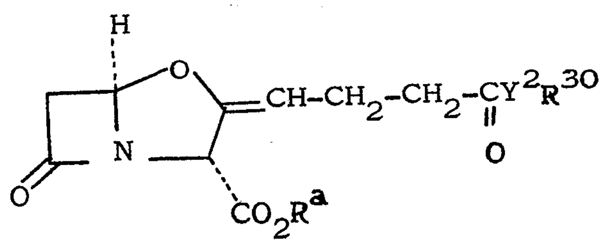

(XVI)

and salts and esters thereof wherein $Y^2$, $R^a$ and $R^{30}$ are as hereinbefore defined; which process comprises the reduction of a compound of the formula (XVII):

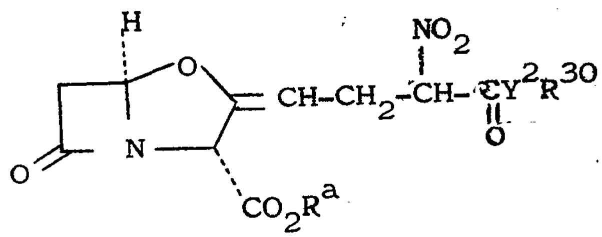

(XVII)

wherein $R^a$ is a hydrogen atom or carboxy-blocking group, $Y^2$ and $R^{30}$ are as hereinbefore defined, and thereafter if necessary:

i) removing any carboxy-blocking group $R^a$,

ii) converting the product into a salt or ester.

Preferably $Y^2$ is an oxygen atom.

Suitably the reduction is performed using a tin hydride reducing agent such as a triaryl tin hydride, di-alkyl tin hydride or trialkyl tin hydride. Suitable aryl groups include phenyl and optionally substituted phenyl. Suitable alkyl groups include $C_{1-6}$ alkyl groups and especially n-butyl. Preferred reducing agents are triphenyl tin hydride and tri-n-butyl tin hydride.

This reaction may be carried out in the presence of a radical initiator, such as di-t-butyl peroxide and azobisisobutyronitrile.

The reaction is generally operated in an inert solvent, such as benzene, toluene, and saturated hydrocarbons. Halogenated hydrocarbon solvents and ethers are not so suitable. Once initiated the reaction is fairly facile, however, the reaction mixture normally requires heating to about 80°C before much reaction occurs. At lower temperatures the reaction may be initiated by using a radical initiator, or photochemically.

The compounds of the formula (XVII) may be prepared by the reaction of either a compound of the formula (XVIII) or (XIX):

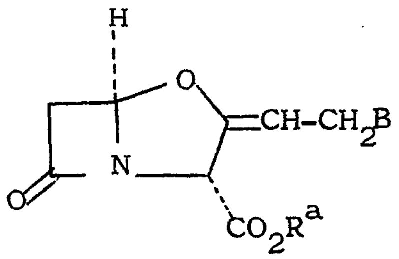

(XVIII)

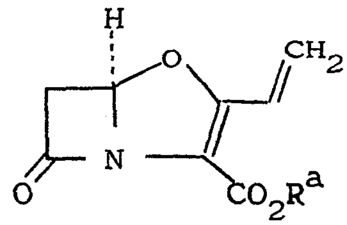

(XIX)

wherein $R^a$ is as hereinbefore defined and B is a leaving group, with a compound of the formula (XX):

$$NO_2\text{-}CH_2\text{-}\underset{\underset{O}{\|}}{C}Y^2R^{30} \qquad (XX)$$

in the presence of a base. Suitably the base is inorganic and capable of forming the carbanion of the compound of the formula (XX), for example a suitable base is sodium hydride.

Suitably B is a carboxylate (OCOQ) moiety wherein Q is an organic group. More suitably

Q is $C_{1-6}$ alkyl, benzyl or dichloromethyl.

The compounds of the formulae (XVIII) and (XIX) may be prepared by the methods described in British Patent Specification Numbers 1529434, 1534508 and 1570495.

Example 1

Benzyl 9-O-(dimethoxymethyl) clavulanate

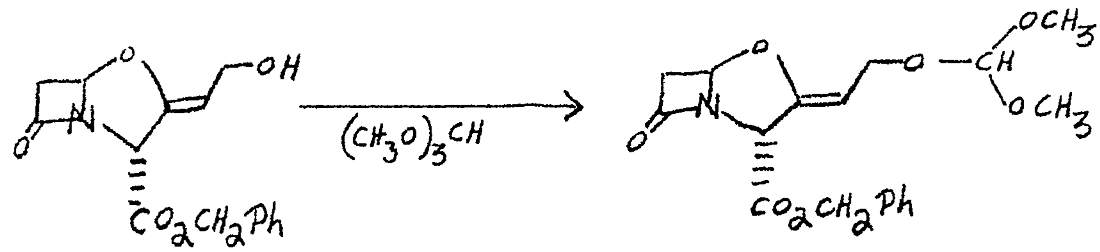


Benzyl clavulanate (2.89g; 10 mmole) was dissolved in trimethylorthoformate (15ml) and warmed to 35°C while being placed under vacuum (14 mm Hg). After 4 hours the reaction mixture was evaporated to an oil which was chromatographed on silica gel (toluene/ethyl acetate 4:1 as eluent) to give the title ester in 90% yield, 3.27g.

I.R. (film) 1800, 1749 and 1693 $cm^{-1}$.

N.M.R. ($CDCl_3$) δ 3.01(1H, d, *J* 17Hz, 6β-CH), 3.32(6H, s, $OCH_3$ x 2), 3.52(1H, dd, *J* 17 and 3 Hz, 6α-CH), 4.17(2H, d, *J* 8Hz, 9-$CH_2$), 4.88(1H, m, 8-CH), 5.08(2H, m, 3-CH and $(MeO)_2$ C<u>H</u>), 5.2(2H, s, $CH_2Ph$), 5.7(1H, d, *J* 3Hz, 5-CH) and 7.39(5H, s, aromatics).

Example 2

Benzyl 2(2-bromoethoxy)-2-vinyl-clavam-3-carboxylate

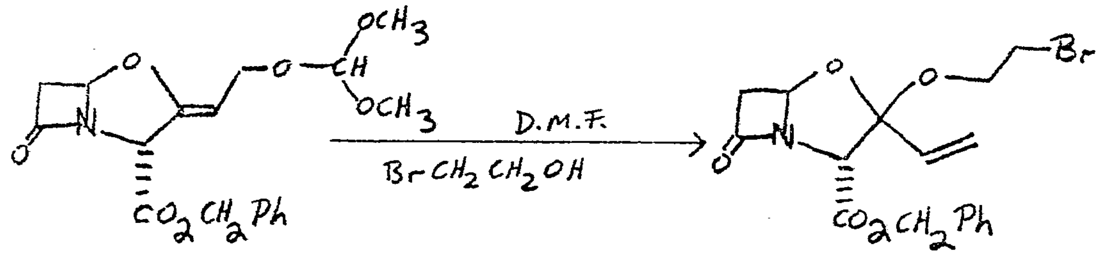


A freshly prepared sample of Benzyl 9-O-(dimethoxymethyl) clavulanate formed from 1.4g of Benzylclavulanate was, without purification, dissolved in dry dimethylformamide (5ml) and 2-bromoethanol (3.633g, 29mmol) was then added. The reaction vessel was placed under vacuum (14 m.m.Hg) and warmed to 40°C. After a reaction time of 2.5 hours the mixture was diluted with ethylacetate and washed with brine. The organic layer was dried ($MgSO_4$) and evaporated prior to silica gel chromatography (Toluene/Ethyl acetate 10:1 as eluent) to leave the bromide as a crystaline solid (from diethylether or ethanol) in 26% yield, 0.498g, and Benzyl clavulanate was recovered in 20% yield, 0.28g.

I.R. (KBr) 1780 and 1751 $cm^{-1}$.

N.M.R. ($CDCl_3$) δ, 3.07(1H, dd, J 16 and 1.5Hz, 6β-CH), 3.36(3H, m, 6α-CH and $OCH_2CH_2Br$), 3.74(2H, m, $OCH_2CH_2Br$), 4.75(1H, s, 3-CH), 5.04(2H, s, $CH_2Ph$), 5.22(1H, q, J 10 and 4 Hz, CH:$CH_2$), 5.64(3H, m, 5-CH and C:$CH_2$) and 7.32(5H, s, aromatics).

Example 2A

Benzyl 2-(2-bromoethoxy)-2-vinyl-clavam-3-carboxylate (improved method)

Benzyl clavulanate (30 g, 0.104 mol) was dissolved in triethyl orthoformate (50 ml) containing a trace of p-toluenesulphonic acid. The reaction was placed under vaccum (14 mm Hg) and occasionally warmed to 30$^{o}$C. The reaction time was variable (circa 6 hr.) but when t.l.c. indicated the consumption of starting material the reaction was evaporated. The residual oil was taken up into 2-bromoethanol (25 ml) and placed under vacuum (10 mm Hg). After 6-10 hr. the reaction mixture was diluted with diethyl ether and washed with a saturated sodium hydrogen carbonate solution (5x). The ether layer was dried ($MgSO_4$) and filtered through Celite before evaporation. The residue was chromatographed on silica gel (toluene/ethylacetate, 7:1 as eluent) affording the title product as a crystalline solid from ethanol, 11.5 g (28% yield).

Example 3

Benzyl 2-(2-chloroethoxy)-2-vinyl-clavam-3-carboxylate

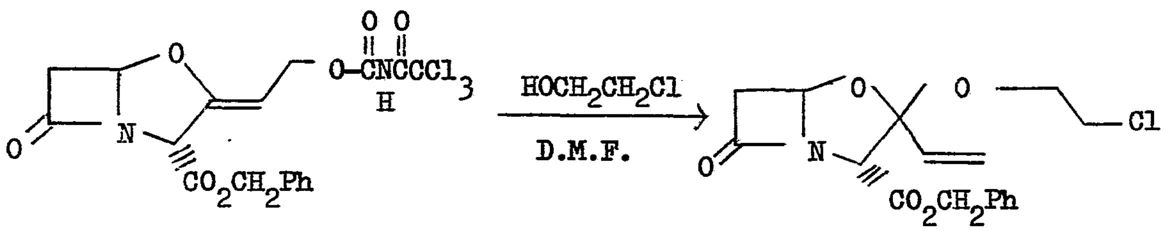


A solution of benzyl 9-(N-trichloroacetyl)-carbamoylclavulanate (17.68g, 37mmol) in dry dimethylformamide ( 5ml) and 2-chloroethanol (20ml, 149mmol) was heated to 55°C for 6 hours. At the end of this time the reaction mixture was diluted with ethyl acetate and washed with brine (3x), saturated sodium hydrogen carbonate solution and water (3x). The organic phase was dried ($MgSO_4$) and evaporated to an oil which was chromatographed upon silica gel (toluene/ethyl acetate 8:1 as eluent) to afford the title ester as a crystalline solid, 2.138g (16.4% yield).

I.R. (Nujol) 1786 and 1755 $cm^{-1}$.

N.M.R. ($CDCl_3$) δ 2.90-3.88(6H, m, 6-$CH_2$ and $OCH_2CH_2Cl$). 4.77(1H, s, 3-CH), 5.05(2H, s, $CH_2Ph$), 5.23(1H, q, J 9 and 4Hz, C<u>H</u>:$CH_2$), 5.5-5.8(3H, m, 5-CH and CH:C<u>H</u>$_2$), 7.35(5H, s, aromatics).

Example 4

Benzyl 2(2-bromoethoxy)-2-vinyl-clavam-3-carboxylate

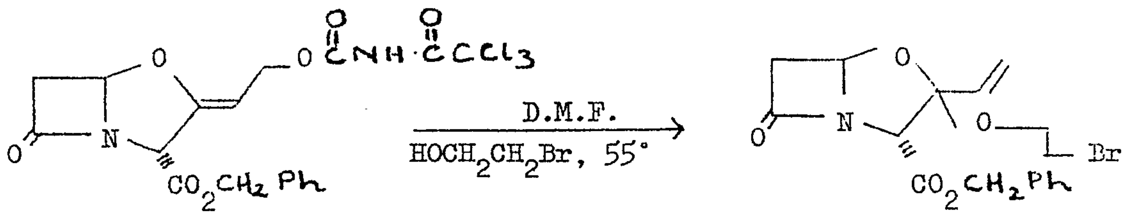


Benzyl 9(N-trichloroacetyl) carbamoylclavulanate formed from Benzylclavulanate (11.53g; 39.9mmol) and trichloroacetyl isocyanate (5.68ml; 47.88mmol) in dichloromethane (100ml), was concentrated and subsequently dissolved in dry dimethylformamide (10ml). 2-Bromoethanol (8.6ml, 119.7mmol) was added to the solution and the reaction was heated to 50°C. After a reaction time of 6-8 hours the mixture was diluted with ethylacetate and washed with brine and saturated sodiumhydrogen carbonate solution. The organic phase was dried ($MgSO_4$) and evaporated to an oil from which the title ester could be crystalized (from ethanol) or isolated by silica gel chromatography (Toluene/ethylacetate, 9:1 as eluent), 4.38g 28% yield.

Example 5

Benzyl 2-(2-benzeneselenoethoxy)-2-vinyl-clavam-3-carboxylate

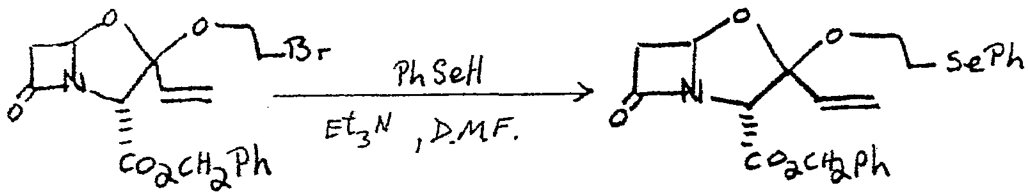


Benzyl 2(2-bromoethoxy)-2-vinyl-clavam-3-carboxylate (3.77g, 9.5 mmol) was dissolved in dimethylformamide (15 ml) and cooled to 0°C. Benzeneselenol (1.2 ml, 11.36 mmol) was added and subsequently triethylamine (1.46 ml, 10.49 mmol) in dimethylformamide (2ml) was added dropwise over 5 minutes. After 1 hour the reaction was diluted with ethyl acetate and washed with brine and dilute hydrochloric acid. The organic layer was dried ($MgSO_4$) and evaporated prior to silica gel chromatography (Toluene/Ethyl acetate 7:1 as eluent) to give the title ester in 80.1% yield, 3.5826g.

I.R. (film) 1800 and 1750 $cm^{-1}$.

N.M.R. ($CDCl_3$) δ, 3.01(4H, m, 6-$CH_2$ and $OCH_2CH_2Se$), 3.64(2H, m, $OCH_2CH_2Se$), 4.7(1H, s, 3-CH), 5.04(2H, s, $CH_2Ph$), 5.22(1H, q, J 9 and 4 Hz, C<u>H</u>:$CH_2$), 5.58(3H, m, 5-CH and C:$CH_2$), and 7.32(10H, m, aromatics).

Example 5a

Benzyl 2-(2-benzeneselenoethoxy)-2-vinyl-clavam-3-carboxylate

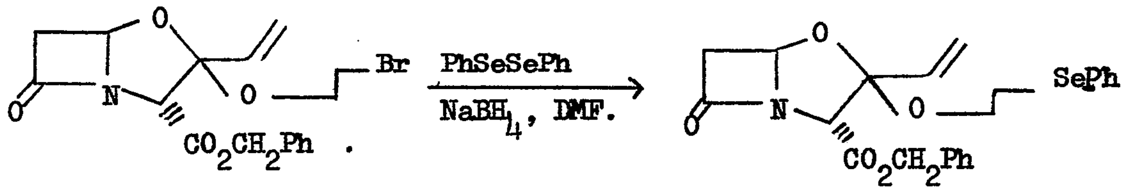


Benzyl 2-(2-bromoethoxy)-2-vinyl-clavam-3-carboxylate (0.396g, 1 mmol) was dissolved in dry dimethylformamide, placed under a stream of nitrogen, and cooled to -20°C. Over the next 5 minutes a dry dimethylformamide solution of diphenyldiselenide (0.172g, 0.55 mmol) and sodium borohydride (0.0416g, 1.1 mmol) was added dropwise. Cooling was halted when this addition was complete and the reaction vessel was allowed to warm to ambient temperature. After 15 minutes at this temperature the reaction mixture was diluted with ethyl acetate and washed with brine (2x), dilute hydrochloric acid and saturated sodium hydrogen carbonate solution. The organic phase was separated and dried ($MgSO_4$) prior to evaporation and fractionation upon silica gel (toluene/ethyl acetate as eluent, 7:1). The title ester was thus obtained in 70% yield (0.33g).

Example 6

Benzyl 2-(2-benzeneselenoethoxy)-2-vinyl-clavam-3-carboxylate.

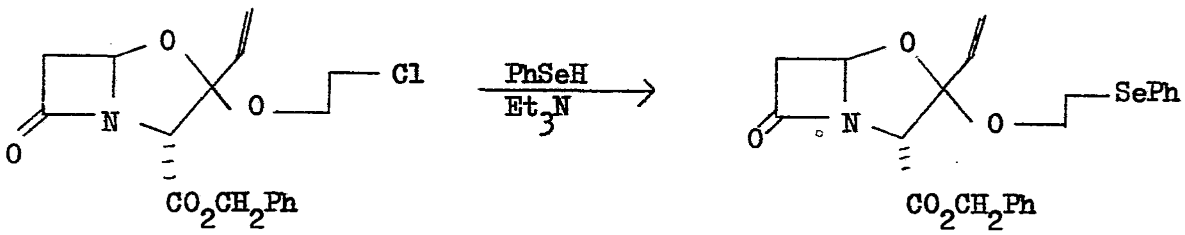


Benzyl 2-(2-chloroethoxy)-2-vinyl-clavam-3-carboxylate (4.28144g; 12.2 mmol) in dry toluene (25ml) was treated with benzeneselenol (1.54ml, 14.64 mmol). Then triethylamine (1.785 ml, 12.81 mmol) was added dropwise. After 6-8 hours the reaction was diluted with ethyl acetate and washed with brine (3x) and saturated sodium hydrogen carbonate solution. The organic layer was separated and dried ($MgSO_4$) prior to silica gel chromatography (toluene/ethyl acetate, 12:1 as eluent). The title ester was obtained in 88% yield as a colourless syrup.

Example 7

Benzyl 2-(2-benzeneseleninylethoxy)-2-vinyl-clavam-3-carboxylate

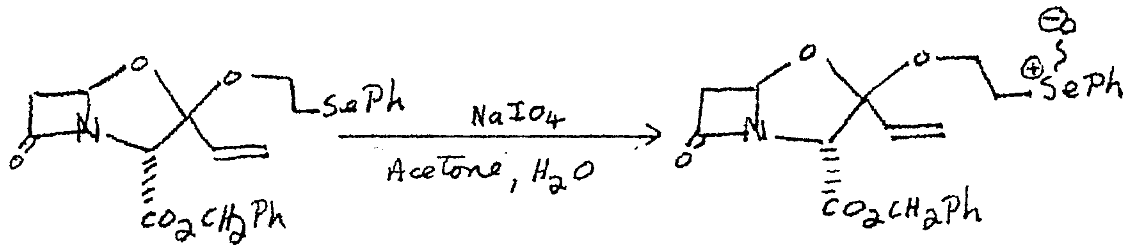


Benzyl 2-(2-benzeneselenoethoxy)-2-vinyl-clavam-3-carboxylate (3.0446g, 6.45 mmol) was dissolved in acetone (20ml). To this solution was added finely ground sodiumperiodate as a suspension in water (5ml). After 15 minutes the reaction mixture was diluted with ethyl acetate and washed with brine (2x) and saturated sodium hydrogen carbonate solution. The organic layer was dried ($MgSO_4$) and evaporated to a foam, 95% yield 2.99g.

I.R. (film) 1799 and 1748 $cm^{-1}$.

N.M.R. ($CDCl_3$) δ 3.2(4H, m, 6-$CH_2$ and $OCH_2CH_2Se$), 3.76(2H, m, $OCH_2CH_2Se$), 4.61 and 4.67(each, 1H, s, 3-CH), 5.05(2H, s, $CH_2Ph$), 5.22(1H, m, C<u>H</u>:$CH_2$), 5.56(3H, m, 5-CH and C:$CH_2$) and 7.47(10H, m, aromatics).

Example 7A

Benzyl 2-(2-benzeneseleninylethoxy)-2-vinyl-clavam-3-carboxylate

Benzyl-2 (2-benzeneselenoethoxy)-2-vinyl-clavam-3-carboxylate (10 g, 21.2 mmol) in acetone (100 ml), at 0°C, was treated with sodium periodate (10 g, 46.7 mmol) in water (25 ml). When t.l.c. indicated that the starting material had been consumed (circa 30 min) the reaction mixture was filtered and concentrated to half volume, diluted with ethylacetate and washed with sodium hydrogen carbonate solution (3x) and brine (2x). The organic layer was dried ($MgSO_4$) and evaporated to an oil which was crystalised from toluene, 9.97 g (96% yield).

0080286

Example 8

Benzyl 2-(4-oxobutylidene)-clavam-3-carboxylate

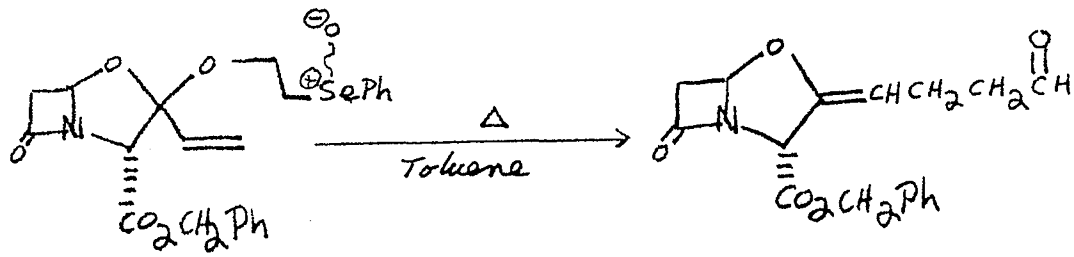


Benzyl 2-(2-benzeneseleninylethoxy)-2vinyl-clavam-3-carboxylate (2.99g, 6.13 mmol) was dissolved in dry toluene (50 ml) and heated to reflux. Twenty minutes after the start of reflux the reaction was cooled and evaporated. The oily residue was chromatographed on silica gel (Toluene/Ethyl acetate 9:1 as eluent) to afford the title ester in 60% yield, 1.93g.

I.R. (film) 1800, 1748, 1728 and 1698 cm $^{-1}$.

N.M.R. ($CDCl_3$) δ, 2.33 (4H, m, $CH_2CH_2$), 2.94 and 2.98(each 1H, d, *J* 16Hz, 6β-CH), 3.41 and 3.45(each 1H, dd, *J* 16 and 2Hz, 6α-CH), 4.54 and 4.94(each 1H, m, 8-CH), 4.99 and 5.23(each 1H, m, 3-CH), 5.15(2H, s, $CH_2Ph$), 5.63(1H, m, 5-CH), 7.32(5H, s, aromatics), 9.5 and 9.66(each 1H, s, aldehyde).

Example 9

Benzyl 2-(4-hydroxybutylidene)-clavam-3-carboxylate

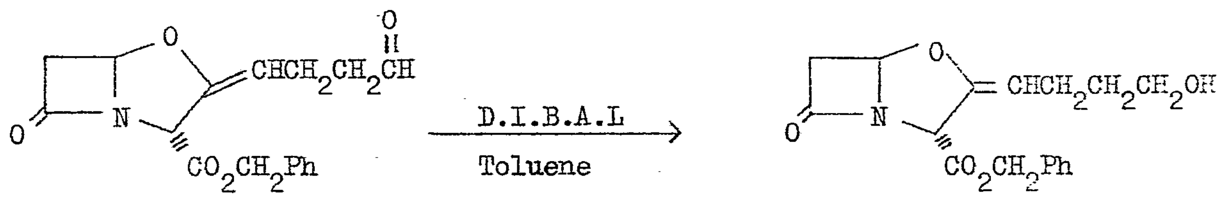


Benzyl 2-(4-oxobutylidene)-clavam-3-carboxylate (0.5g, 1.587mmol) was dissolved in dry toluene (10ml) and cooled to -20°C while under dried nitrogen gas. Di-isobutylaluminiumhydride (2.104ml of a 25% solution in toluene) was added dropwise. After 2 hours the viscous reaction mixture was emptied into ethyl acetate which was washed with dilute hydrochloric acid. The emulsion thus formed was filtered through 'celite' before the separation of the organic phase which was dried ($MgSO_4$) and evaporated. The residue was chromatographed on silica gel (toluene/ethyl acetate 2:1 as eluent) affording the Z and E isomers of the title ester in 13.5 and 12% yield respectively (0.034g and 0.030g).

E isomer, I.R. (film) 3,430, 1800, 1747 and 1697 $cm^{-1}$.

N.M.R. ($CDCl_3$) δ 1.53(2H, m, $CH_2C\underline{H}_2CH_2$), 1.74(1H, br s, OH), 2.01(2H, m, C:CHC$\underline{H}_2$), 3.99(1H, d, *J* 17Hz, 6β-CH), 3.49(3H, m, 6α-CH and C$\underline{H}_2$OH), 5.02(1H, dt, *J* 8 and 1Hz, 8-CH), 5.2(2H, s, $CH_2$ Ph), 5.26(1H, br s, 3-CH), 5.66(1H, d, *J* 3Hz, 5-CH), 7.39(5H, s, aromatics). [Addition of $D_2O$ caused the br s at δ 1.74 to disappear].

Z isomer, I.R. (film) 3420, 1801, 1745 and 1700 $cm^{-1}$.

N.M.R. ($CDCl_3$) δ, 1.65(2H, m, $CH_2C\underline{H}_2CH_2$), 1.78(1H, br s, OH), 2.2(2H, m, C:CHC$\underline{H}_2$), 3.05(1H, d, *J* 18Hz, 6α-CH), 3.54(3H, m, 6β-CH and C$\underline{H}_2$OH), 4.61(1H, dt, *J* 8 and 1Hz, 8-CH), 5.07(1H, br s, 3-CH), 5.23(2H, s, $CH_2$Ph), 5.69(1H, d, *J* $2H_3$,5-CH)

and 7.41(5H, s, aromatics). [Addition of $D_2O$ caused the br s at δ 1.78 to disappear].

Example 10

Benzyl E-2-(4-oxobutylidene)-clavam-3-carboxylate

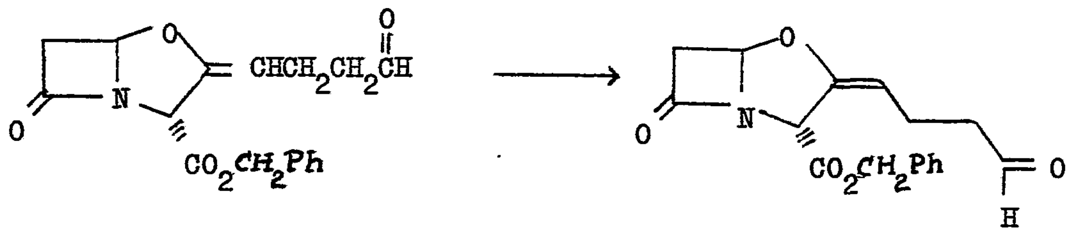

Benzyl 2-(4-oxobutylidene)-clavam-3-carboxylate (2g, 6.35mmol) as a 50:50 mixture of Z and E isomers was cooled to -20°C and diethylether, previously cooled to -20°C, was added. The mixture was scratched as a solution was formed. The E isomer crystallizes out as a white solid. The solution was kept at -20°C for 4 hours at which time it was filtered and the white solid washed with cold diethylether. The crystalline E isomer was obtained in 75% yield (0.75g) and the residual oil remains as a mixture of Z amd E isomers (8:2). This process may be repeated to obtain the E isomer in 90% yield (0.9g) leaving the residual oil as a 9:1 mixture of Z to E isomers.

E isomer: I.R. (Nujol) 1789, 1743, 1715 and 1694 $cm^{-1}$.

N.M.R. ($CDCl_3$) δ 2.27(4H, m, $C\underline{H}_2C\underline{H}_2$), 2.94(1H, d, $\underline{J}$17Hz, 6-βC$\underline{H}$), 3.45(1H, dd, $\underline{J}$ 17 and 3Hz, 6-α C$\underline{H}$), 5.02(4H, m, 3C$\underline{H}$, 8C$\underline{H}$ and C$\underline{H}_2$Ph), 5.61(1H, d, $\underline{J}$2Hz, 5C$\underline{H}$), 7.33 (5H, s, aromatics) and 9.57(1H, s, CO.$\underline{H}$).

Example 11

Benzyl 2-(4-hydroxybutylidene)-clavam-3-carboxylate

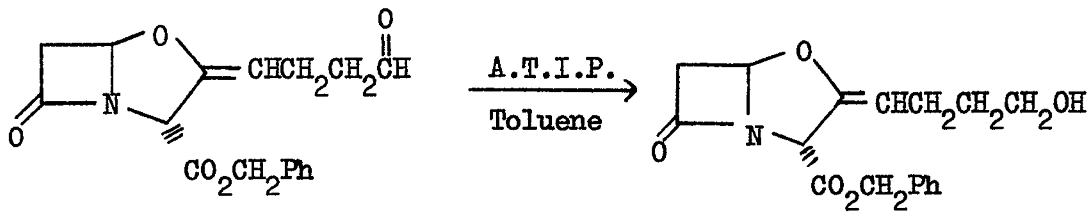


Benzyl 2-(4-oxobutylidene)-clavam-3-carboxylate (0.5g, 1.587 mmol) was dissolved in dry toluene (20 ml). To this solution was added aluminium tri-iso-propoxide (0.324g, 1.587 mmol) portionwise. The homogenous solution was heated to 60°C to distil off acetone and after 20 minutes the reaction mixture was cooled and diluted with ethyl acetate, which was washed with dilute hydrochloric acid and saturated sodium hydrogen carbonate solution. The organic phase was dried ($MgSO_4$) and evaporated prior to silica gel chromatography (toluene/ethyl acetate 2:1 as eluent) to afford the Z and E isomers of the title ester in 12 and 11% yield respectively (0.03g and 0.0275g).

Example 12

Benzyl 2-(4-hydroxybutylidene)-clavam-3-carboxylate

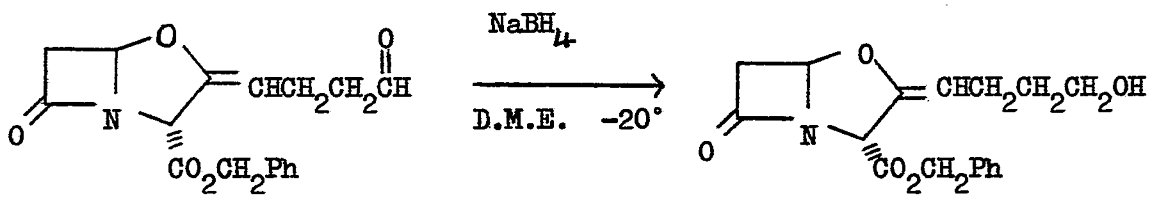


Benzyl 2-(4-oxobutylidene)-clavam-3-carboxylate (0.8583g, 2.725mmol) as an 8:2 mixture of Z to E isomers was dissolved in dry dimethoxyethane (10ml) and cooled to -20°C. Sodium borohydride (0.124g, 3.278mmol) was added portion-wise over 5 minutes and the reaction was kept at -20°C for a further 15 minutes, by this time the starting material had been consumed as was judged from monitoring by t.l.c. The reaction mixture was diluted with ethyl acetate and washed with brine (2x), dilute hydrochloric acid and finally sodium hydrogen carbonate solution before drying ($MgSO_4$) the organic layer. Evaporation of this layer and silica gel fractionation of the residue (toluene/ethyl acetate 2:1 as eluent) afforded both the E isomer (0.1123g, 65% yield) and the Z isomer (0.5372g, 78% yield) of the title ester.

Example 13

Benzyl E-2-(4-hydroxybutylidene)-clavam-3-carboxylate

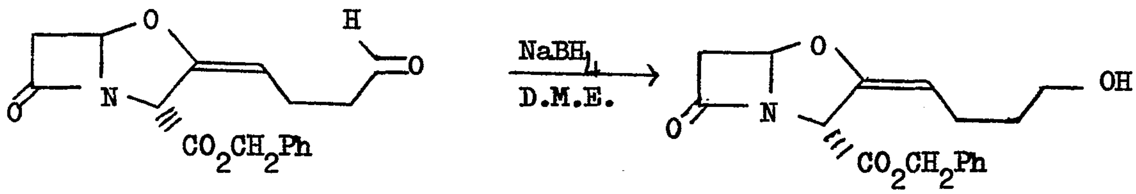


Sodium borohydride (0.062g, 1.64mmol) was added portion-wise to a solution of benzyl E-2-(4-oxobutylidene)-clavam-3-carboxylate (0.43g, 1.365mmol) in dry dimethoxyethane cooled to -20°C. After 20 minutes the reaction mixture was diluted with ethyl acetate and washed with brine (2x), dilute hydrochloric acid and saturated sodium hydrogen carbonate solution. The organic phase was dried ($MgSO_4$) and evaporated to an oil which was chromatographed on silica gel (toluene/ethyl acetate, 2:1 as eluent) to afford the title ester, 0.2814g (65% yield).

Example 14

Lithium 2-(4-hydroxybutylidene)-clavam-3-carboxylate

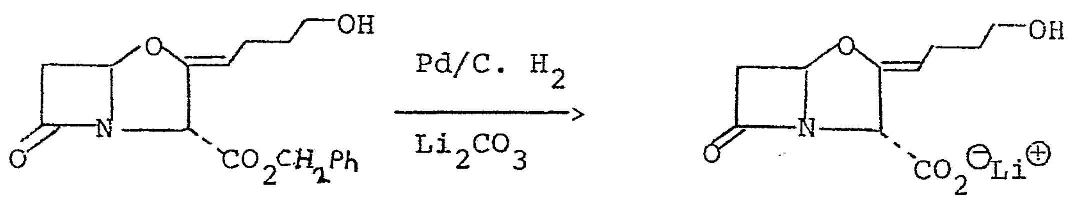


Benzyl 2(Z)-(4-hydroxybutylidene)-clavam-3-carboxylate (0.028g, 0.0883mmol) in tetrahydrofuran (2ml) was added to prehydrogenated palladium on carbon (10%, 12mg) in aqueous (1ml) tetrahydrofuran (3ml) containing lithium carbonate (0.00327g, 0.0442mmol). The reaction was hydrogenated for 20 minutes by which time the reaction was complete as judged by T.L.C. The catalyst was filtered off and washed with water; the filtrate and washings were combined and evaporated to leave the product as a solid, 9 mg, 43.7% yield.

I.R. (Nujol) 3,400, 1770, 1700 and 1623 $cm^{-1}$. N.M.R. $\delta$ ($D_2O$) 1.62 (2H, quintet, J7 and 8Hz, $OCH_2\underline{CH_2}$), 2.14 (2H, ddt, J8Hz, 8Hz and 0.7Hz, C:$CH\underline{CH_2}$), 3.075 (1H, d, J18Hz, 6-β CH), 3.535 (1H, dd, J2 and 18Hz, 6-α CH), 3.585 (2H, t, 7Hz, $\underline{CH_2}OH$), 4.71 (1H, dt, J8 and 1Hz, C:CH), 4.875 (1H, br. s, 3-CH), 5.665 (1H, d, J2Hz, 5-CH.)

Example 15

Lithium Z-2-(4-hydroxybutylidene)-clavam-3-carboxylate

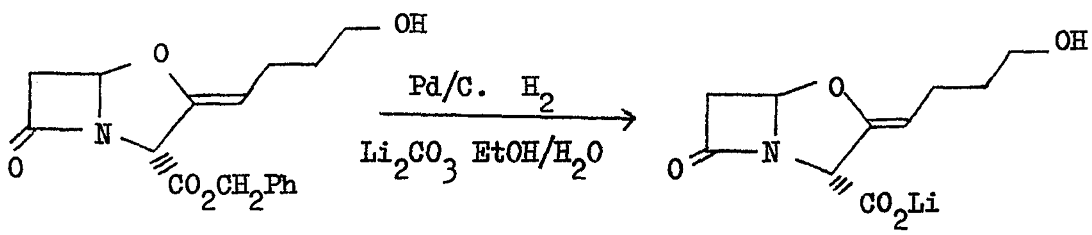


Benzyl Z-2-(4-hydroxybutylidene)-clavam-3-carboxylate (0.5372g, 1.695 mmol) in ethanol (10ml) was added to palladium on carbon (0.2g; 10%) and lithium carbonate (0.0626g, 0.847 mmol) in water (5ml). The reaction mixture was hydrogenated for 15-20 mins. by which time the starting material had been consumed. The catalyst was filtered off and washed with ethanol which was combined with the filtrate and concentrated under vacuum. Addition of acetone to the concentrated aqueous solution caused the title salt to crystallize. Filtration and drying of the solid afforded pure lithium Z-2-(4-hydroxybutylidene)-clavam-3-carboxylate (0.2675g, 67.7% yield).

I.r. (Nujol) 3570, 3320, 1784, 1700 and 1623 $cm^{-1}$.

0080286

Example 16

Lithium E-2-(4-hydroxybutylidene)-clavam-3-carboxylate

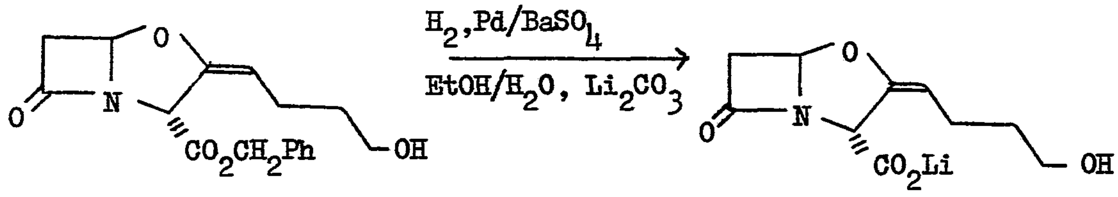


Benzyl E-2-(4-hydroxybutylidene)-clavam-3-carboxylate (0.2814g, 0.888mmol) in ethanol (8ml) was added to palladium on bariumsulphate (0.15g, 5%) and lithium carbonate (0.0328g, 0.444 mmol) in water (3ml). The reaction mixture was hydrogenated for 20 minutes by which time the starting material had been consumed. The catalyst was filtered off and washed with ethanol which was combined with the filtrate and concentrated under vacuum. Acetone was added to the concentrated aqueous solution causing the title salt to crystallize out. Filtration and drying of the solid afforded pure lithium E-2-(4-hydroxybutylidene)-clavam-3-carboxylate (0.1012g, 49% yield).

I.R. (Nujol) 3490, 1759, 1690 and 1620 $cm^{-1}$.

N.M.R. ($D_2O$) δ 1.55(2H, m, $CH_2C\underline{H}_2CH_2$), 2.01(2H, m, $C{:}CHC\underline{H}_2$), 2.96(1H, d, J17Hz, 6-βC$\underline{H}$), 3.49(3H, m, 6-αC$\underline{H}$ and C$\underline{H}_2$OH), 4.98 (2H, m, 3C$\underline{H}$ and 8C$\underline{H}$), 5.57(1H, d, J2Hz, 5C$\underline{H}$).

Example 17

Benzyl 2-[(3-ethoxycarbonyl-3-nitromethyl)propylidene]-clavam-3-carboxylate

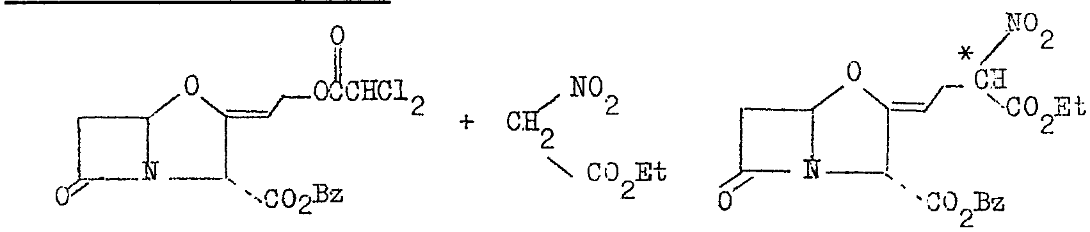

Sodium hydride in oil (480 mg, 50% dispersion in oil; 10mmol) was added portionwise to a solution of ethyl nitroacetate (2.0g; 15mmol) in dry redistilled dimethylformamide and the resulting suspension stirred for 20 minutes. A solution of the benzyl dichloroacetate (4g; 10mmol) in dry dimethylformamide was added and the reaction mixture was stirred for 30 minutes at 0° and for 4 hours at room temperature. A red solution resulted; this was poured into ethyl acetate (300ml), washed with water (2 x 100ml), saturated brine (1 x 100ml) and dried (anhydrous $MgSO_4$). The solvent was evaporated to give an oil which was chromatographed on silica gel eluting with cyclohexane - methyl acetate, 2 : 1. Fractions were collected containing the title epimeric compound Rf ($SiO_2$/cyclohexane - methylacetate, 2 : 1) = 0.37, (detection by aqueous potassium permanganate). Yield 40%.

$\nu_{max.}$ (film) 1795, 1740, 1695 $cm^{-1}$. $\delta$ ($CDCl_3$) 1.29 (3H, t, J7Hz, $CH_2\underline{CH_3}$), 2.97 (2H, m, 9$\underline{CH_2}$), 3.09 (1H, bd, J17Hz, 6β-$\underline{CH}$), 3.5 (1H, dd, J17 and 3Hz, 6α-$\underline{CH}$), 4.27 (2H, two pairs of overlapping quartets, J7Hz, $\underline{CH_2}CH_3$), 4.6 (1H, bd, J7Hz, 8-$\underline{CH}$), 5.04 (1H, bs, 3-$\underline{CH}$), 5.09, 5.13 (1H, 2 x dd, J5 and 3Hz, $\underline{CH}$ ($NO_2$)·$CO_2CH_2CH_3$) 5.15, 5.21 (2H, ABq, J12Hz, $\underline{CH_2}Ar$), 5.69, 5.72 (1H, 2 x d, J3Hz, 5-$\underline{CH}$) 7.38 (5H, m, Ar-$\underline{H}$).

Example 18

Benzyl 2-[3-benzyloxycarbonyl-3-nitromethyl)propylidene]-clavam-3-carboxylate

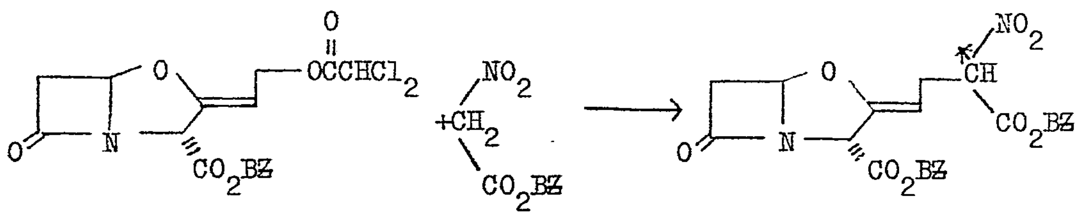

Benzyl nitroacetate (5.85g 30mmol) in dry redistilled dimethylformamide (35ml) was treated with sodium hydride (960mg, 50% dispersion in oil; 20mmol) at 0°; the suspension was stirred for ~ 10 minutes. A solution of benzyl dichloroacetylclavulanate (8g; 20mmol) in dry redistilled dimethylformamide (15ml) was added and the mixture was stirred for 30 minutes at 0° and for four hours at room temperature. The reaction mixture was poured into ethylacetate (300ml) and washed with water (2 x 100ml) and saturated brine (2 x 100ml) dried (anhydrous $MgSO_4$) and evaporated to an oil. Fractionation on silica gel, eluting with cyclohexane-ethylacetate, 3:1, gave the product as a colourless oil 3.3g (35% yield) Rf ($SiO_2$/cyclohexane-ethylacetate, 2:1)= 0.26 (detection by aqueous potassium permanganate).

$\nu$ ($CHCl_3$) 1803, 1752, 1698 $cm^{-1}$, $\delta$ ($CDCl_3$) 2.99(2H, m, 9-C$\underline{H}_2$) 3.05(1H, d, J 17.5Hz, 6β-C$\underline{H}$), 3.46(1H, dd, J 17.5 and 3Hz, 6α-C$\underline{H}$) 4.56, 4.58(1H, 2 x dt, J 7 and 1.5Hz, 8-C$\underline{H}$), 5.02, 5.04(1H, 2 x d, J 1.5Hz, 3-C$\underline{H}$), 5.15(1H, m, C$\underline{H}$ ($NO_2$)$CO_2$Bz, partly obscured by s at $\delta$ 5.15), 5.15(2H, s, C$\underline{H}_2$Ar), 5.22, 5.24(2H, 2 x s, C$\underline{H}_2$ Ar), 5.63, 5.66(1H, 2 x d, J 3Hz, 5-C$\underline{H}$) 7.35(10H, m, Ar-$\underline{H}$).

0080286

Example 19

Benzyl 2-[(3-ethoxycarbonyl)propylidene]-clavam-3-carboxylate

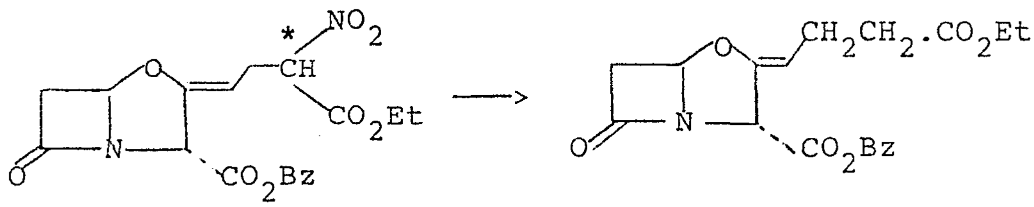

The product of Example 17 (480mg; 1.2mmol) was treated under nitrogen with tributyltin hydride (379ml. 1.4mmol) in refluxing toluene (50ml) in the presence of azabisisobutyronitrile (39mg; 0.24mmol); the reaction was monitored by t.l.c. After ≃ 4 hours the solvent was evaporated in vacuo and the resultant oil chromatographed on silica gel with toluene-ethylacetate, 7:1 as the eluent. The product was obtained as a colourless oil (yield 42%).

$\nu_{max.}$ (film) 1795, 1740 (b), 1695 $cm^{-1}$. δ ($CDCl_3$) 1.25 (3H, t, J7.0Hz, $CH_2\underline{CH_3}$), 2.35 (4H, m, $\underline{CH_2CH_2}$.$CO_2CH_2CH_3$), 3.05 (1H, dd, J17 and 1.5Hz, 6β-C$\underline{H}$), 3.47 (1H, dd, J17 and 3Hz, 6α-C$\underline{H}$), 4.12 (2H, q, J7.0Hz, $\underline{CH_2}CH_3$), 4.62 (1H, bt, 7Hz, 8-CH), 5.03 (1H, bs, 3-$\underline{CH}$), 5.15, 5.21 (2H, ABq, J11.5Hz, $\underline{CH_2}$Ar) 5.66 (1H, d, J3Hz, 5-$\underline{CH}$), 7.37 (5H, m, Ar-$\underline{H}$).

Example 20

Lithium 2-[(3-ethoxycarbonyl)propylidene]-clavam-3-carboxylate

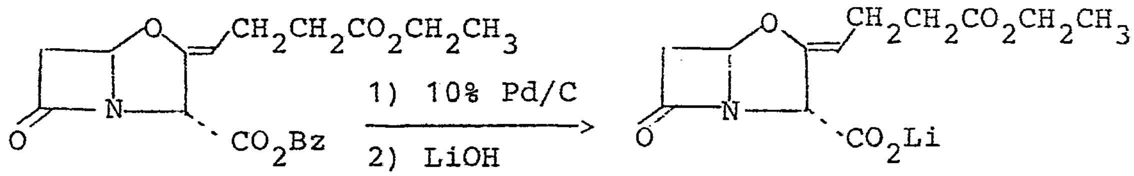


The product of Example 19 (120mg; 0.33mmol) in dry redistilled tetrahydrofuran (25ml) was hydrogenated over 10% Pd/C (60mg) for 40 minutes. The catalyst was filtered off and water (10ml) was added to the filtrate which was titrated to pH 7.3 with 0.1M lithium hydroxide. The aqeuous solution was evaporated to dryness and the product crystallised from acetone. Yield 51%.

$\nu_{max.}$ (KBr) 1760 - 1790 (b), 1720 (b), 1618 $cm^{-1}$.
$\delta$ ($D_2O$) 1.22 (3H, t, J7.5Hz, $CH_2\underline{CH_3}$), 2.42 (4H, m, $\underline{CH_2}\underline{CH_2}CO_2CH_2CH_3$), 3.07 (1H, d, J17Hz, 6β-$\underline{CH}$), 3.53 (1H, dd, J17 and 3Hz, 6α-$\underline{CH}$), 4.12 (2H, q, J7.5Hz, $\underline{CH_2}CH_3$), 4.68 (1H, bt, J7.5Hz, 8-$\underline{CH}$), 4.86 (1H, bs, 3-$\underline{CH}$), 5.66 (1H, d, J3Hz, 5-$\underline{CH}$).

Example 21

Benzyl 2-[4-(methylsulphonyloxy)butylidene)]-clavem-3-carboxylate.

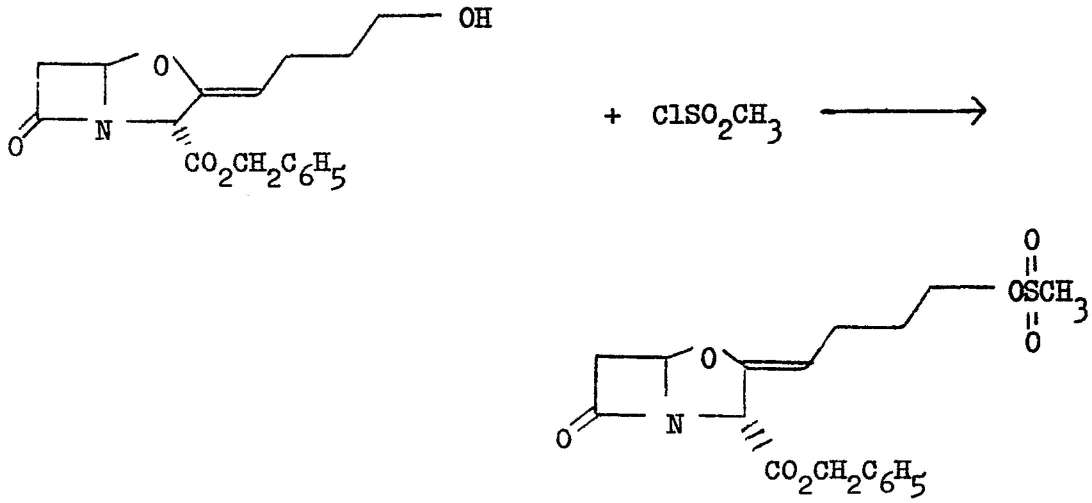


Benzyl 2-(4-hydroxybutylidene)-clavam-3-carboxylate (408mg; 1.29mmol) in dry dichloromethane (6cm$^3$) at -30° was treated with 1 equivalent of pyridine followed by 1 equivalent of methanesulphonylchloride, dropwise. The temperature was allowed to rise to room temperature when a further 0.2cm$^3$ of pyridine and 0.1cm$^3$ (1 equivalent) of methanesulphonyl chloride was added. The mixture was stirred for 72 hours when thin layer chromatography showed the absence of starting material.

The reaction mixture was diluted with dichloromethane (50cm$^3$) and washed with saturated brine, dried (anhydrous magnesium sulphate) and evaporated to an oil. This oil was chromatographed on silica eluting with toluene-ethylacetate; 3:1, fractions were collected containing the required product, Rf ($SiO_2$/toluene-ethylacetate; 3:1) = 0.5 (detection by aqueous potassium permanganate spray).

Combined fractions were evaporated to afford an oil, yield = 0.47g (92%).

ν (5% in $CHCl_3$) 1800, 1750, 1700, 1358, 1335, 1305, 1172, 1120, 1000, 970, 955, 932$cm^{-1}$ δ($CDCl_3$) 1.68-1.83(2H, m, $CH_2C\underline{H}_2CH_2O$), 2.20(2H, broad dt, *J* 7 and 7Hz, 2'$C\underline{H}_2$), 2.98 (3H, s, $SC\underline{H}_3$), 3.07(1H, broad d, *J* 17Hz, 6βC$\underline{H}$), 3.47(1H, dd, *J* 17 and 2.5Hz, 6αC$\underline{H}$), 4.07-4.21(2H, m, $CH_2CH_2C\underline{H}_2O$), 4.53(1H, dt, *J* 7 and 1Hz, 1'C$\underline{H}$), 5.04(1H, d, *J* 1Hz, 3C$\underline{H}$), 5.16 and 5.25(2H, ABq, *J* 12Hz, $CO_2C\underline{H}_2$), 5.66(1H, broad d, *J* 2.5Hz, 5αC$\underline{H}$), 7.31-7.43(5H, m, $C_6\underline{H}_5$).

Example 22

Benzyl 2-[4-(p- toluenesulphonyloxy)butylidene]-clavam-3-carboxylate.

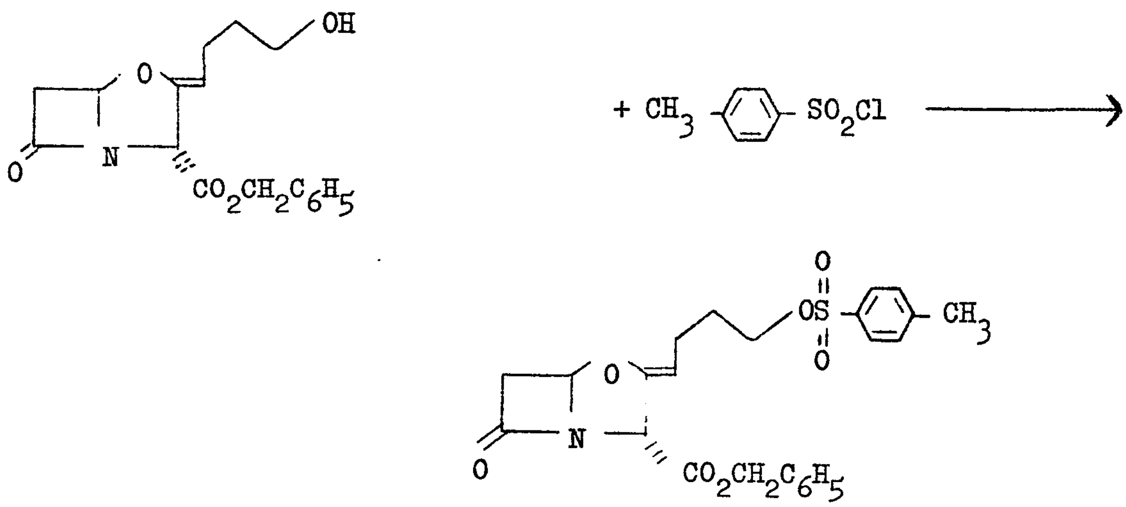

Benzyl 2-(4-hydroxybutylidene)-clavam-3-carboxylate (0.81g; 2.56mmol) in dry methylene dichloride ($12cm^3$) at -30°C was treated with 1 equivalent of pyridine, followed by 1.1 equivalents of p -toluene sulphonyl chloride in methylene dichloride ($5cm^3$).

The mixture was stirred and the temperature allowed to rise to 20°C over ½ hour, when a further equivalent of pyridine was added. After 24 hours, ½ equivalent of p -toluenesulphonyl chloride was added and a further equivalent of pyridine. Stirring was continued for a further 24 hours when thin layer chromatography (t.l.c.) still showed unreacted starting material present. A further ½ equivalent of tosyl chloride was added and the reaction mixture allowed to stand at 5° for a further 48 hours when t.l.c. showed the absence of starting material. The reaction mixture was diluted with methylene dichloride and washed with saturated brine (5 x $5cm^3$), dried (anhydrous magnesium sulphate) and evaporated to an oil.

This crude oil was chromatographed on silica eluting with toluene-ethyl acetate (4:1) and fractions were collected containing the required product, Rf ($S;O_2$; toluene: ethyl acetate, 4:1)=0.56 (detection by aqueous potassium permanganate spray). Combined fractions were evaporated to an oil, yield = 0.90g (75%) ν (film) 1805, 1752, 1700 $cm^{-1}$ ν (5% in $CHCl_3$) 1800, 1750, 1700, 1360, 1307, 1175, 1000, 968, 935 $cm^{-1}$. δ ($CDCl_3$) 1.44-1.80(2H, m, $CH_2CH_2OSO_2$), 2.11(2H, dt, J 7 and 7Hz, $CH_2CH_2CH_2OSO_2$), 2.41(3H, s, $CH_3$), 2.98(1H, d, J 16.5Hz, 6βCH), 3.41(1H, dd, J 16.5 and 2.5Hz, 6 α CH), 3.92(2H, t, J 6Hz, $CH_2OSO_2$), 4.43(1H, broad t, J 7Hz, 1'CH), 4.97(1H, broad s, 3CH), 5.15(2H, s, $CO_2CH_2$), 5.60(1H, d, J 2.5Hz, 5 α CH), 7.18-7.45(7H, m, $C_6H_5$ and 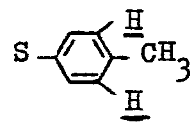 ), 7.75(2H, $d_{AB}$, J 8Hz, 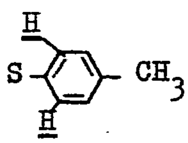 ).

Example 23

Benzyl 2-(4-azidobutylidene)-clavam-3-carboxylate

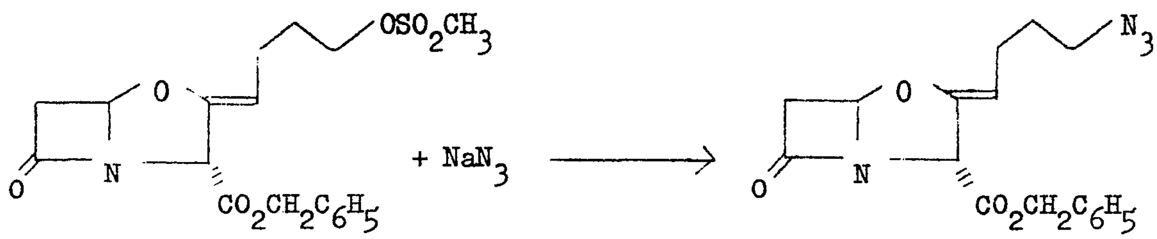

Benzyl 2-[4-(methylsulphonyloxy)butylidene]-clavem-3-carboxylate (175mg) in dimethylformamide (4cm$^3$) at 20° was treated with 2 equivalents of sodium azide and stirred for 6 hours. The mixture was diluted with ethyl acetate (100cm$^3$) and washed with saturated brine (5 x 50cm$^3$), dried (anhydrous magnesium sulphate) and evaporated to an oil.

The oil was chromatographed on silica eluting with ethyl acetate-toluene; 1:6. Fractions were collected and fractions containing a material Rf 0.6 were combined and evaporated ($SiO_2$/ethyl acetate-toluene; 1:6) to an oil, yield = 30mg.

ν (film) 2090, 1802, 1750, 1697, 740, 698 cm$^{-1}$
δ ($CDCl_3$) 1.51-1.67(2H, m, $CH_2C\underline{H_2}CH_2N_3$), 2.16(2H, ddt, $\underline{J}$ 7, 1 and 7Hz, $C\underline{H_2}CH_2CH_2N_3$), 3.05(1H, broad d, $\underline{J}$ 16Hz, 6β$C\underline{H}$), 3.18(2H, t, $\underline{J}$ 7Hz, $C\underline{H_2}N_3$), 3.47(1H, dd, $\underline{J}$ 16 and 2.5Hz, 6α$C\underline{H}$), 4.53(1H, dt, $\underline{J}$ 7 and 1Hz, 1'$C\underline{H}$), 5.04(1H, bd, $\underline{J}$ 1Hz, 3$C\underline{H}$), 5.16 and 5.25(2H, ABq, $\underline{J}$ 12Hz, $CO_2C\underline{H_2}$), 5.67(1H, broad d, $\underline{J}$ 2.5Hz, 5α$C\underline{H}$), 7.30-7.45(5H, m, $CH_2C_6\underline{H_5}$).

Example 24

Benzyl 2-(4-azidobutylidene)-clavam-3-carboxylate

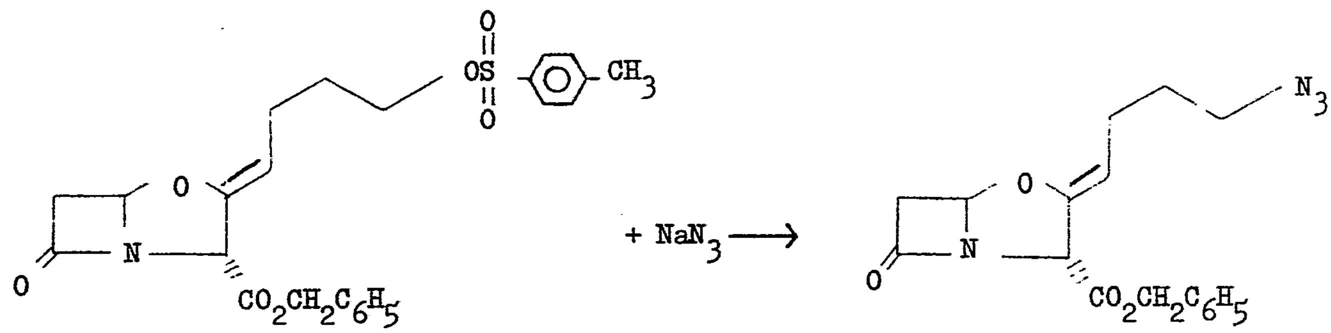

A solution of benzyl 2-(4-p-toluenesulphonyloxybutylidene)-clavam-3-carboxylate (0.75g; 1.6 mmol) in dimethylformamide (10 $cm^3$) at room temperature was treated with two equivalents of sodium azide and stirred for 4¾ hours. The mixture was poured into ethyl acetate (150 $cm^3$) and washed with saturated brine (5 x 50 $cm^3$), dried (anhydrous magnesium sulphate) and evaporated to an oil. This oil was chromatographed on silica eluting with toluene-ethyl acetate (8:1). Fractions were collected containing the title compound, Rf ($SiO_2$/toluene-ethyl acetate; 6:1) = 0.6 and combined fractions were evaporated to afford 250 mg (45%) of an oil; ν (film) 2090, 1803, 1750, 1695, 740, 698, $cm^{-1}$.

Example 25

2-(4-Aminobutylidene)-clavam-3-carboxylate

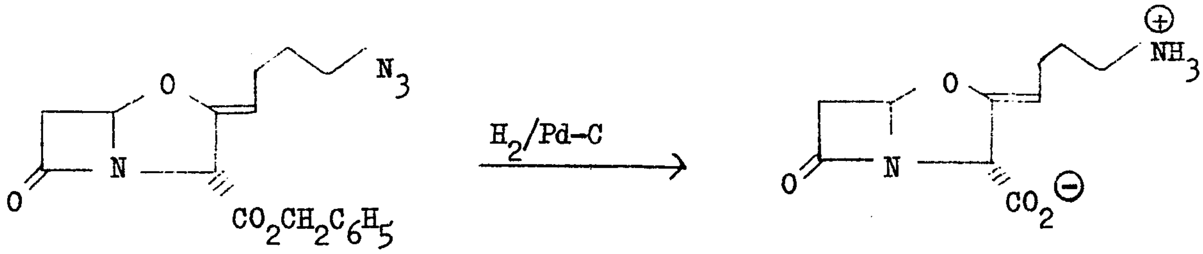


Benzyl 2-(4-azidobutylidene)-clavam-3-carboxylate (34.0mg; 0.1mmol) in tetrahydrofuran ($3cm^3$) was hydrogenolysed at atmospheric pressure in the presence of 10% palladium on carbon (20mg) for 2½ hours. The catalyst was filtered off and washed with tetrahydrofuran ($10cm^3$), then with water ($20cm^3$). The aqueous washings were collected separately and evaporated to afford a white crystalline solid from ethanol.

Yield = 10mg, ν (nujol) 1797, 1690, 1638, 1585 $cm^{-1}$
δ ($D_2O$; HOD at δ 4.80) 1.67-1.81(2H, m, $CH_2C\underline{H}_2CH_2N$), 2.12-2.28 (2H, m, 2'$C\underline{H}_2$), 2.98(2H, t, J 7Hz, $C\underline{H}_2\overset{\oplus}{N}H_3$), 3.07(1H, d, J 16Hz, 6βC$\underline{H}$), 3.53(1H, dd, J 16 and 3 Hz, 6 α C$\underline{H}$), 4.68(1H, broad t, J 7Hz, 1'C$\underline{H}$), 4.89(1H, broad s, 3C$\underline{H}$), 5.68(1H, d, J 3Hz, 5C$\underline{H}$).

Example 26

Benzyl 2-(4-methoxybutylidene)-clavam-3-carboxylate

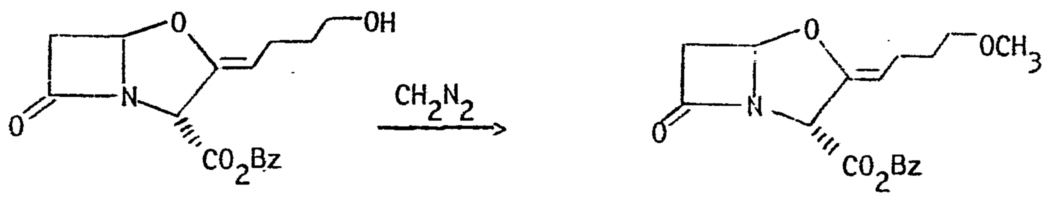


To a solution of benzyl-2-(4-hydroxybutylidene) clavam-3-carboxylate (0.525 g; 1.7 mmol) in dichloromethane (5 ml), at 0°C, was added diazomethane in diethyl ether (70 ml; approx. 0.4 m solution) and a catalytic amount of borontrifluoride etherate. The reaction mixture was allowed to stand at 0°C for 8 hr. Nitrogen was bubbled through the solution until it was colourless and then ethylacetate (20 ml) was added. The organic phase was washed with sodium bicarbonate solution (2 x 50 ml), brine (2 x 50 ml) and finally dried over anhydrous magnesium sulphate. Removal of the solvent in vacuo gave a page yellow oil; which was subjected to silica gel column chromatography eluting with ethyl acetate : cyclohexane (2 : 3) to give the title compound (0.36 g; 92%) Rf = 0.44 (ethylacetate : cyclohexane; 2 : 3) and recovered starting alcohol (0.15) Rf = 0.16 (ethyl acetate : cyclohexane, 2 : 3) both as colourless oils.

$\nu_{max.}$ ($CHCl_3$) 1790, 1740 and 1690 $cm^{-1}$.

$\delta_H$ ($CDCl_3$) 1.59 (2H, m, :$CHCH_2\underline{CH_2}CH_2OCH_3$), 2.12 and 2.16 (2H, dt, J 7 Hz, :$CH\underline{CH_2}CH_2CH_2OCH_3$), 3.02 (1H, broad d, J 16 Hz, 6β-$\underline{CH}$), 3.32 (3H, s, $O\underline{CH_3}$), 3.32 (1H, t, J 7 Hz, :$CHCH_2CH_2\underline{CH_2}OCH_3$), 3.45 (1H, dd, J 16 and 2.5 Hz, 6α-$\underline{CH}$), 4.57 (1H, dt, J 7 and 1 Hz, :$\underline{CH}CH_2CH_2CH_2OCH_3$), 5.03 (1H, d, J 1 Hz, 3-$\underline{CH}$), 5.24 and 5.16 (2H, ABq, J 12 Hz, $\underline{CH_2}Ph$), 5.65 (1H, brad d, J 2.5 Hz, 5-$\underline{CH}$), 7.23 - 7.45 (5H, m, Ar$\underline{H}$).

Example 27

Sodium 2-(4-methoxybutylidene)-clavam-3-carboxylate

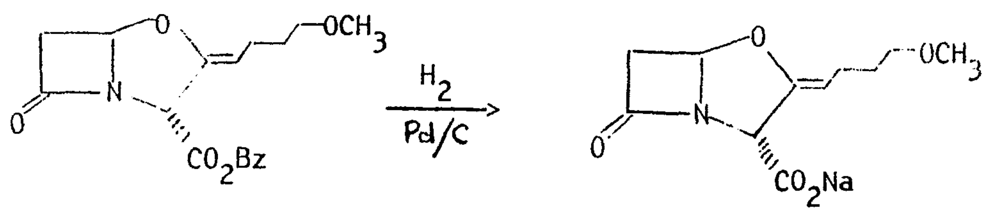


Benzyl 2-(4-methoxybutylidene)-clavam-3-carboxylate (0.35 g; 1.05 mmol) in 5% aqueous tetrahydrofuran (15 ml) was hydrogenated for 20 min. at room temperature over 10% palladium on carbon (0.12 g). Thin layer chromatography indicated that all of the starting material had been consumed. The reaction mixture was filtered and reduced to a small volume (approx. 1 ml) then partitioned between ethyl acetate (15 ml) and water (15 ml). Adjustment of the pH to 7.2 with sodium hydroxide solution (1 M) followed by separation and evaporation of the aqueous layer yielded the desired salt as a pale yellow gel which on addition of ether gave a solid (0.28 g; 93%).

$\nu_{max.}$(KBr) 1785, 1695 and 1610 $cm^{-1}$.

$\delta_H$ ($D_2O$) 1.63 (2H, H, :$CHCH_2\underline{CH_2}CH_2OCH_3$) 2.12 (2H, m, :$CH\underline{CH_2}CH_2CH_2OCH_3$) 3.06 (1H, d, J 16 Hz, 6β-$\underline{CH}$), 3.32 (3H, S, $O\underline{CH_3}$), 3.45 (2H, t, J 7 Hz, :$CHCH_2\underline{CH_2}CH_2OCH_3$), 3.52 (1H, dd, J 16 and 2.5 Hz, 6α-$\underline{CH}$), 4.69 (1H, dt, J 7 and 1 Hz, :$\underline{CH}CH_2CH_2CH_2OCH_3$), 4.86 (1H, d, J 1 Hz, 3-$\underline{CH}$), 5.65 (1H, d, J 2.5 Hz, 5-$\underline{CH}$).

Example 28

Benzyl 2-(4-methoxybutylidene)clavam-3-carboxylate

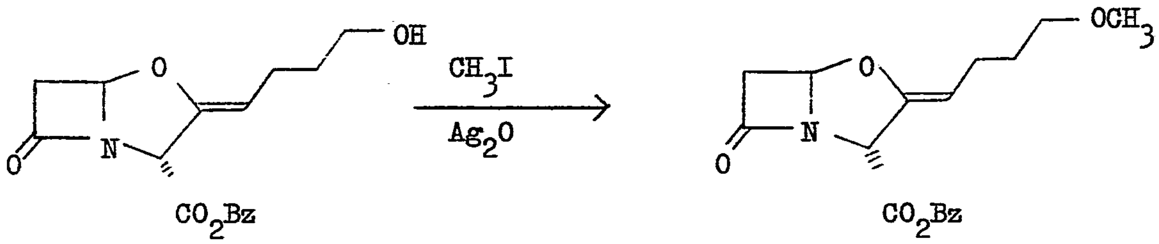


Benzyl 2-(4-hydroxybutylidene)clavam-3-carboxylate (0.317g; 1mmol); silver (I) oxide (0.23g); calcium sulphate (0.32g) and methyl iodide (0.65ml; 0.4mmol) were stirred together in dichloromethane (5ml) in a stoppered flask in the dark for 48 hours. Thin layer chromatographic analysis indicated that approximately 50% of the starting material had been consumed. The reaction mixture was filtered and a fresh quantity of the reagents (methyl iodide; 0.65ml, silver oxide; 0.23g, calcium sulphate; 0.32g) added and the mixture was stirred for a further 15 hours, then filtered, and the solvent removed under reduced pressure to yield a yellow oil which was subjected to silica gel column chromatography eluting with ethyl acetate: toluene (1:10). The fractions containing component Rf 0.46 (ethyl acetate: toluene; 1:7) were combined and evaporated *in vacuo* to give the desired ether (80mg; 25%) as a colourless oil.

$\nu_{max}$ (5% $CHCl_3$) 1790, 1740, and 1690 $cm^{-1}$

N.M.R. ($CHCl_3$) same as previous sample.

0080286

Example 29a

Benzyl 2-(2-benzenethioethoxy)-2-vinyl-clavam-3-carboxylate.

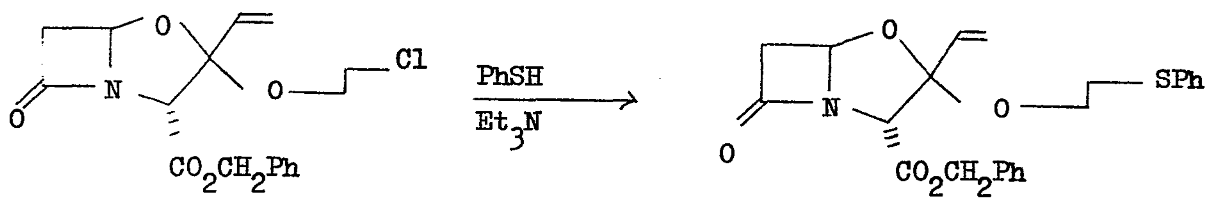


Benzyl 2-(2-chloroethoxy)-2-vinyl-clavam-3-carboxylate (1.2834g, 3.02mmol) in dry dimethylformamide (10ml) was treated sequentially with thiophenol (0.6ml) and triethylamine (0.6ml). After 6 hours at room temperature the reaction was diluted with ethyl acetate and washed with brine (3x), water (3x) dilute hydrochloric acid (2x) and finally saturated sodium hydrogen carbonate solution. After drying and evaporation the residue was columned upon silica gel (toluene/ethyl acetate, 9:1 as eluent) affording the title ester in 76.6% yield (1.192g).

$\nu_{max}$ (film) 1800 and 1749 $cm^{-1}$.
$\delta$ ($CDCl_3$) 2.70-3.80 (6H, m, O-$CH_2CH_2$-s and β-lactam $CH_2$), 4.67 (1H, s, C3-H), 4.98(2H, s, $CH_2Ph$), 5.13(1H, q, J 8 and 4Hz, CH: $CH_2$), 5.41-5.70(3H, m, $C_5$-H and CH:$CH_2$), 6.96-7.40(10H, m, aromatics).

Example 29b

Benzyl 2-(2-benzenesulfinylethoxy)-2-vinyl-clavam-3-carboxylate

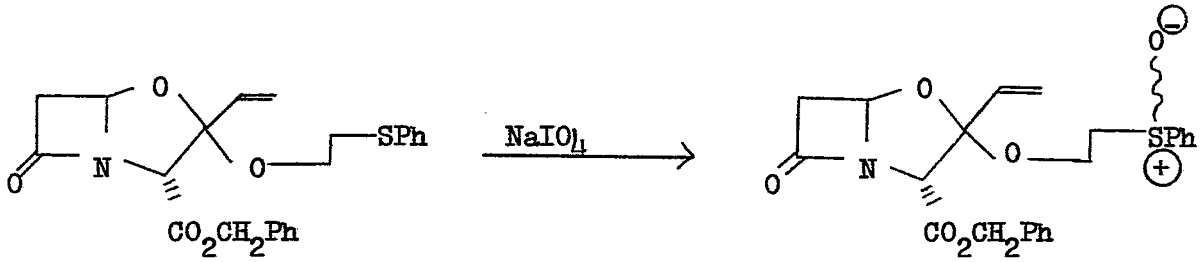


Benzyl 2-(2-benzenethioethoxy)-2-vinyl-clavam-3-carboxylate (0.8047g, 1.89 mmol) in aqueous acetone (20ml acetone/5ml water) was treated with finely ground sodium metaperiodate (1g). The reaction was allowed to stir at ambient temperature for two days by which time the reaction was complete as judged from thin layer chromatography. The reaction was diluted with ethyl acetate and washed with brine (3x) and saturated sodium hydrogen carbonate solution before evaporation and silica gel chromatography (toluene/ethyl acetate 2:1 as eluent) to give the title ester in 73.3% yield (0.612g).

$\nu_{max}$ (nujol) 1793, 1742 $cm^{-1}$.
δ ($CDCl_3$) 2.62-4.10 (6H, m, $O.CH_2.CH_2.S$ and β-lactam $CH_2$), 4.69 and 7.79 (1H, each d, J < 2Hz, C3-H for each S ∼ O isomer), 5.04(2, s, $CH_2Ph$), 5.13-5.43(1H, m,CH:$CH_2$), 5.50-5.81(3H, m, CH:$CH_2$ and C5-H), 7.21-7.76(10H, m, aromatics).

Example 29c

Benzyl 2-(4-oxobutylidene)-clavam-3-carboxylate

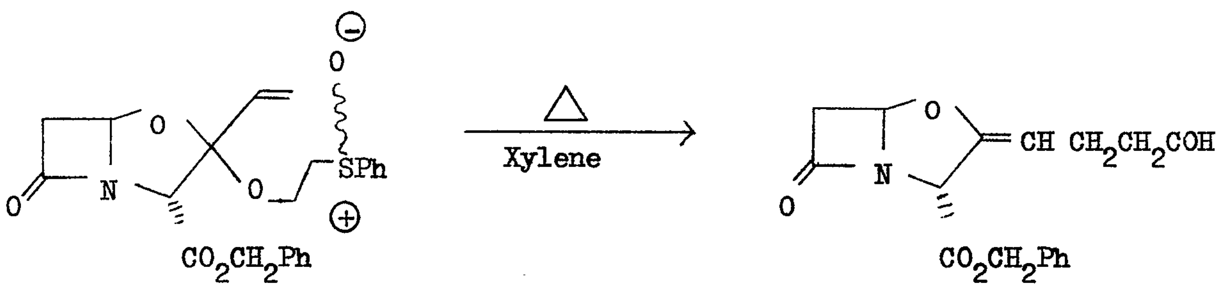


Benzyl 2-(2-benzenesulfinylethoxy)-2-vinyl-clavam-3-carboxylate (0.432g, 0.979 mmol) in dry xylene (20ml) was refluxed for 36 hours. By this time all starting material had been consumed and the reaction mixture was evaporated and the residue subjected to silica gel chromatography (toluene/ethyl acetate 7:1 as eluent). The title ester was thus obtained in 5% yield (0.0155g).

Example 30

Benzyl 2-[4-(5-ethoxycarbonyltetrazol-1-yl) butylidene] clavam-3-carboxylate.

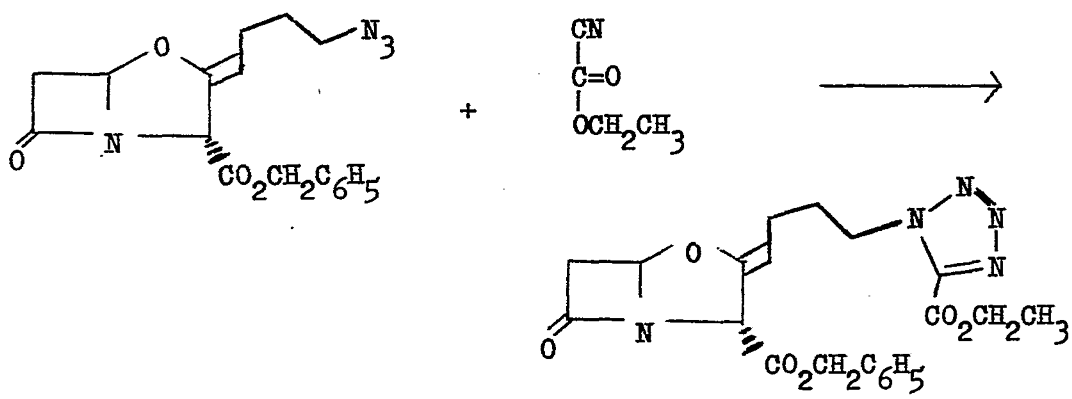

Benzyl 2-(4-azidobutylidene) clavam-3-carboxylate (180mg; 0.53mmol) in neat ethylcyanoformate (2 $cm^3$) was stirred at 85° for 21 hours when a further 1.5 $cm^3$ ethylcyanoformate was added. The reaction was stirred for a further 23 hours at 80° and 65 hours at 70°. Thin layer chromatography showed starting material still present. A further 1 $cm^3$ ethylcyanoformate was added and the reaction continued for 61 hours at 80° (total 170 hours). The ethylcyanoformate was evaporated off under vacuum and the residue chromatographed on silica eluting with ethyl acetate: toluene; 1:6. Fractions were collected containing the title compound Rf ($SiO_2$/ethyl acetate-toluene; 1:6) = 0.30 (detection by aqueous potassium permanganate spray). Combined fractions were evaporated to afford an oil, yield = 117mg (50%) $\nu$ (film) 1803, 1743, 1700 $cm^{-1}$; 250 MHz FT $^1H$ n.m.r. $\delta$ ($CDCl_3$) 1.49 (3H, t, $J$ 7Hz, $CH_2C\underline{H}_3$), 1.90-2.05 (2H, m, $CH_2C\underline{H}_2CH_2N$), 2.14 (2H, dt, $J$7 and 7Hz, $C\underline{H}_2CH_2CH_2N$), 3.07 (1H, d, $J$ 17Hz, 6$\beta$C$\underline{H}$), 3.47(1H, dd, $J$17 and 3Hz, 6$\alpha$C$\underline{H}$), 4.53(2H, q, $J$7Hz, $C\underline{H}_2CH_3$), 4.55(1H, dt, $J$ 1 and 7Hz, =<$\underline{H}$ $CH_2$), 4.64(2H, t, $J$ 7Hz, $CH_2C\underline{H}_2N$), 5.04(1H, d, $J$1Hz, 3C$\underline{H}$), 5.17(1H) and 5.25(1H) ABq, $J$12Hz, $OC\underline{H}_2C_6H_5$), 5.65(1H, d, $J$3Hz, 5 $\alpha$ C$\underline{H}$), 7.34(5H, s, $CH_2C_6\underline{H}_5$).

Example 31a

Benzyl 4-tetrazol-1-ylbutylidene-clavam-3-carboxylate and benzyl 4-tetrazol-2-ylbutylidene-clavam-3-carboxylate

Benzyl 4-hydroxybutylidene-clavam-3-carboxylate (0.5 g; 1.58 mmol) in dry tetrahydrofuran at 20°C was treated with tetrazole (221 mg; 3.16 mmol) and triphenyl phosphine (497 mg; 1.9 mmol) and cooled to 0°. Diethylazodicarboxylate (330 mg; 1.9 mmol) was added with stirring. After 20 mins. a further 0.2 equivalents of triphenylphosphine was added and 50 μl diethylazodicarboxylate. Stirring was continued for 0.75 hours allowing the temperature to rise to 5°. The solvent was evaporated and the residue dissolved in ethylacetate-cyclohexane (1.2), the resulting crystals were filtered off and the filtrate chromatographed on silica eluting with ethylacetate-cyclohexane 1:2 grading to 1:1. Fractions were collected containing material Rf [$SiO_2$/ ethyl acetate-cyclohexane (1:1)] = 0.75 (detection by aqueous potassium permanganate spray). Combined fractions were evaporated to afford a colourless oil, yield = 275 mg (47%) of the tetrazol-2-yl derivative. ν (film) 1800, 1750, 1700, 1305, 1230, 1180, 1120, 1040, 1025, 1015, 1005, 895, 740, 700 $cm^{-1}$. ν (5% in $CHCl_3$) 1800, 1750, 1700, 1307, 1220, 1180, 1118, 1040, 1015, 1000, 895 $cm^{-1}$: δ ($CDCl_3$) 1.95-2.20 (4H, m, $C\underline{H}_2C\underline{H}_2CH_2$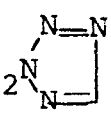), 3.07 (1H, d, $\underline{J}$ 16.5 Hz, 6βC$\underline{H}$), 3.47 (1H, dd, $\underline{J}$ 16.5 Hz and 3 Hz, 6αC$\underline{H}$), 4.45-4.65 (3H, m, 8C$\underline{H}$ and C$\underline{H}_2$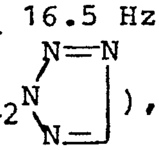), 5.02 (1H, d, $\underline{J}$ 1 Hz, 3C$\underline{H}$), [5.18 (1H, $d_{ABq}$, $\underline{J}$ 11 Hz), 5.25 (1H, $d_{ABq}$, $\underline{J}$ 11 Hz), $OC\underline{H}_2C_6H_5$], 5.65 (1H, broad d, 5αC$\underline{H}$), 7.34 (5H, broad s, $CH_2C_6\underline{H}_5$), 8.49 (1H, s, 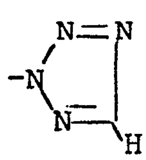).

Further fractions Rf ($SiO_2$/ethyl acetate-cyclohexane 1:1) = 0.3 were combined and evaporated to an oil containing a crystalline solid. This mixture was rechromatographed on silica eluting with ethyl acetate and fractions collected Rf ($SiO_2$/ethyl acetate) ≃ 0.8 (detection by aqueous potassium permanganate spray). Combined fractions were evaporated to afford a colourless oil, (15%) of the tetrazol-1-yl derivative. ν (film) 1800, 1747, 1695, 1305, 1330, 1170, 1115, 1040, 1015, 1000, 960, 890, 742, 700 $cm^{-1}$. ν 5% in $CHCl_3$) 1800, 1745, 1695, 1305, 1220, 1165, 1115, 1040, 1010, 1000 $cm^{-1}$. δ ($CDCl_3$) 1.83-2.14 (4H, broad m, $C\underline{H}_2C\underline{H}_2CH_2$ 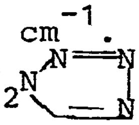 ), 3.04 (1H, broad d, $\underline{J}$ 17 Hz, 6βC$\underline{H}$), 3,475 (1H, dd, $\underline{J}$ 17 and 3 Hz, 6αC$\underline{H}$), 4.10-4.32 (2H, broad m, $C\underline{H}_2$ 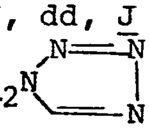 ), 4.48 (1H, dt, $\underline{J}$ 7 and 1 Hz, 8C$\underline{H}$) 5.05 (1H, d, $\underline{J}$ 1 Hz, 3C$\underline{H}$), [5.17, (1H, $d_{AB}q$, $\underline{J}$ 11 Hz), 5.27 (1H, $d_{ABq}$, $\underline{J}$ 11 Hz), $OC\underline{H}_2C_6H_5$], 5.67 (1H, broad d, $\underline{J}$3 Hz, 5αC$\underline{H}$), 7.35 (5H, broad s, $CH_2C_6\underline{H}_5$), 8.48 (1H, s, 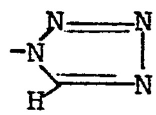 ).

Example 31b

Lithium 4-tetrazol-1'-ylbutylideneclavam-3-carboxylate

Benzyl 4-tetrazol-1'-ylbutylideneclavam-3-carboxylate (225 mg; 0.61 mmol) in tetrahydrofuran (20 $cm^3$) was hydrogenolysed at atmospheric pressure in the presence of 80 mg 10% Pd/C for 70 minutes. The catalyst was filtered off and the filtrate was diluted with distilled water (20 $cm^3$). This solution was triturated to pH 7.2 using 0.2M lithium hydroxide solution. The solvents were evaporated to afford a cream coloured solid from acetone. Yield = 120 mg (70%), Rf ($SiO_2$/ethyl acetate-ethanol-water; 4:2:1) = 0.43 (detection by aqueous potassium permanganate spray). ν (Nujol) 1785, 1685, 1610, 1302, 1170, 1110, 1040, 1015, 995, 895, 735 $cm^{-1}$; ν (KBr) 1782, 1692, 1610, 1408, 1305, 1170, 1115, 1042, 1020, 998, 897, 737 $cm^{-1}$.

δ ($D_2O$) 2.00-2.25 (4H, m, $C\underline{H}_2C\underline{H}_2CH_2$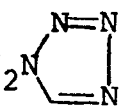 ), 3.09 (1H, d, $\underline{J}$ 17 Hz, 6βC$\underline{H}$), 3.55 (1H, dd, $\underline{J}$ 17 and 3 Hz, 6αC$\underline{H}$), 4.54 (2H, broad t, $\underline{J}$ 6 Hz, $C\underline{H}_2$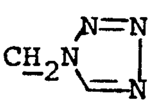 ), 4.66 (1H, t, $\underline{J}$ 8 Hz, $C\underline{H}CH_2CH_2$), 4.86 (1H, s, 3C$\underline{H}$), 5.67 (1H, d, $\underline{J}$ 3 Hz, 5αC$\underline{H}$), 9.18 (1H, s, 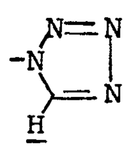 )

Example 32

Lithium 4-tetrazol-2'-ylbutylidene-clavam-3-carboxylate

Benzyl 4-tetrazol-2'-ylbutylidene-clavam-3-carboxylate (540 mg; 1.4 mmol) in tetrahydrofuran (20 $cm^3$) was hydrogenolysed for 70 minutes in the presence of 200 mg 10% palladium on carbon. The catalyst was filtered off and the filtrate diluted to 25 $cm^3$ with tetrahydrofuran and to 50 $cm^3$ with distilled water. This solution was titrated to pH 7.20 using 0.2M lithium hydroxide solution and evaporated to afford a crystalline solid. This solid was washed with acetone and dried. Yield = 330 mg (80%) Rf ($SiO_2$/ ethyl acetate-ethanol-water; 8:4:2) = 0.47 (detection by aqueous potassium permanganate spray). ν (Nujol) 3160 (tetrazol C-H str), 1787, 1690, 1620 (side band), 1605, 1580 (side band), 1308, 1295, 1285, 1190, 1145, 1103, 1070, 1052, 1038, 1030, 1015, 1002, 992, 897, 740 $cm^{-1}$. ν (KBr disc) 3170, 1785, 1690, 1605, 1410, 1305, 1295, 1285, 1187, 1305, 1070, 1055, 1037, 1027, 1015, 1002, 995, 898, 740, 700 $cm^{-1}$.

250M Hz $^1$H n.m.r. δ ($D_2O$) 2.12 (4H, broad s, $C\underline{H}_2C\underline{H}_2CH_2$ 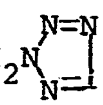 ), 3.06 (1H, d, $\underline{J}$ 17 Hz, 6β$C\underline{H}$), 3.52 (1H, dd, $\underline{J}$ 17 and 3 Hz, 6αCH), 4.58 (1H, t, $\underline{J}$ 7 Hz, $C\underline{H}CH_2CH_2$ 4.65-4.77 (2H, m, $C\underline{H}_2$ 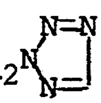 ), 3CH obscured by HOD, 5.62 (1H, d, $\underline{J}$ 3 Hz, 5α$C\underline{H}$), 8.74 (1H, s, 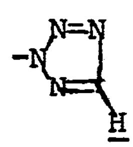 ).

Example 33a

p-Nitrobenzyl 9-(p-toluenesulphonamidocarbonyloxy)-clavulanate

To a solution of p-nitrobenzylclavulanate (25.0g, 74.8 mmol) in dichloromethane (100 ml) at 0°C was added dropwise p-toluenesulphonyl isocyanate (14.8 g, 74.8 mmol) in dichloromethane (30 ml). The reaction was stirred at 0°C for 1 hour, then the solvent removed under reduced pressure to give the title compound as a pale yellow foam (39.2g). $\nu$max ($CHCl_3$) 1810, 1750 and 1700 $cm^{-1}$. $\delta_H$ ($CDCl_3$) 2.41 (3H, s, 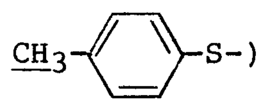),

3.05 (1H, d, J 1.7Hz, 6β-CH), 3.51 (1H, dd, J, 17 and 2Hz, 6α-CH), 4.50-4.85 (3H, m, 9-$CH_2$ and 8-CH),

5.09 (1H, s, 3-CH), 5.25 (2H, s, 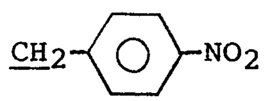),

5.68 (1H, d, J 2Hz, 5-CH), 7.30-7.48 (5H, m, NH,

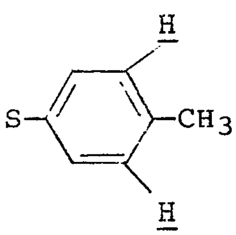 and 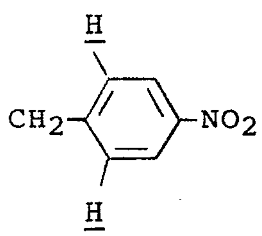), 7.89 and

8.21 (4H, 2 x $d_{AB}$, J9 and 9Hz, 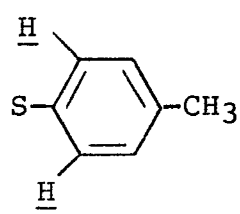 and

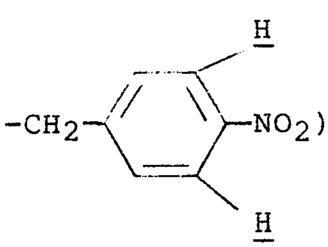).

Example 33b

p-Nitrobenzyl 2-(2-bromoethoxy)-2-vinyl-clavam-3-carboxylate

p-Nitrobenzyl 9-(p-toluenesulphonamido carbonyloxy) clavulanate (12g; 22.6 mmol) was dissolved in 2-bromoethanol (20 ml; 280 mmol) and dimethylformamide (2 ml). The reaction mixture was allowed to stand at 40°C for 16 hours then ethyl acetate (150 ml) added. The organic phase was washed with water (3 x 200 ml) and saturated brine then dried over anhydrous magnesium sulphate. Removal of the solvent gave a yellow solid which was subjected to silica gel clumn chromatography using ethyl acetate:toluene (1:7) as eluent. Fractions containing the component Rf.=0.52 (ethyl acetate: toluene; 1:7) were conbimed and evaporated *in vacuo* to yield the title compound as a white solid, which crystallised from ethanol as colourless needles (24%). $\nu_{max}$ (KBr) 1780 and 1750 $cm^{-1}$. $\delta_H$ ($CDCl_3$) 3.09 (1H, dd, J17 and 1Hz, 6b-CH), 3.22-3.55 (3H, m, 6α-CH and $O-CH_2CH_2Br$), 3.60-3.90 (2H, m, $OCH_2CH_2Br$), 4.79 (1H, s, 3-CH), 5.12 (2H, s, $-CH_2-$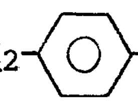$-NO_2$), 5.25 (1H, dd, J9 and 3Hz, $CH:CH_2$), 5.55-5.78 (3H, m, 5-CH and $CH:CH_2$), 7.46 and 8.20 (4H, ABq, J9Hz, ArH).

Example 33c

p-Nitrobenzyl-2-(2-benzeneselenoethoxy)-2-vinyl-clavam-3-carboxylate

Sodium borohydride (0.26 g; 6.9 mmol) was added portionwise to diphenyldiselenide (1.86 g, 5.96 mmol) in dimethylformamide (10 ml) under nitrogen at room temperature. The solution was allowed to stand until colourless then added dropwise over 10 min. to a solution of p-nitrobenzyl 2-(2-bromoethoxy)-2-vinyl-clavam-3-carboxylate (4.05 g; 9.2 mmol) in dimethylformamide (25 ml) at -20°C under nitrogen when the addition was complete the mixture was allowed to warm to room temperature and stirred for a further ¾ hour. The solution was then poured into ethyl acetate 100 ml) and the organic phase washed with saturated brine (2 x 150 ml), dilute hydrochloric acid (150 ml) and saturated sodium bicarbonate solution (150 ml) then dried over anhydrous magnesium sulphate. Evaporation in vacuo gave a yellow oil which was subjected to silica gel column chromatography using ethyl acetate:toluene (1:7) as eluent combinig fractions containing component Rf=0.44 (ethyl acetate:toluene; 1:7) and removing the solvent under reduced pressure gave the selenide as a colourless oil (97%). $\nu$max ($CHCl_3$). 1800 and 1750 $cm^{-1}$. $\delta_H$($CDCl_3$). 2.83-3.12 (3H, m, O-$CH_2$<u>$CH_2$</u>Se and 6β-C<u>H</u>),3.36 (1H, dd, J16 and 3Hz, 6α-C<u>H</u>), 3.50-3.80 (2H, m, O<u>$CH_2$</u>$CH_2$Se), 4.70 (1H, s, 3-<u>CH</u>), 5.10(2H, s, <u>$CH_2$</u>- 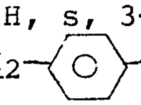 -$NO_2$), 5.19 (1H, dd, J9 and 3Hz, <u>CH</u>:$CH_2$),

5.51-5.75(3H, m, 5-<u>CH</u> and CH:<u>$CH_2$</u>), 7.10-7.60 and 8.20(9H, m, Ar<u>H</u>).

Example 33d

p-Nitrobenzyl 2-(2-benzeneseleninylethoxy)-2-vinyl-clavam-3-carboxylate

p-Nitrobenzyl 2-(2-benzeneselenoethoxy)-2-vinyl-clavam-3-carboxylate (4.3 g; 8.3 mmol) was dissolved in acetone (35 ml) at 0°C and a suspension of sodium metaperiodate (3.5 g; 16.3 mmol) in water (15 ml) added. The reaction mixture was allowed to warm to room temperature and stirred for 1½ hours then poured into ethyl acetate (100 ml). The organic phase was washed with saturated brine (2 x 150 ml), saturated sodium bicarbonate solution (2 x 150 ml) and dried over anhydrous magnesium sulphate. Removal of the solvent yielded the title compound as a colourless oil 4.6 g (97%). $\nu$max ($CHCl_3$) 1800, 1750 and 1610 $cm^{-1}$.

0080286

Example 33e

p-Nitrobenzyl 2-(4-oxobutylidene)clavam-3-carboxylate

p-Nitrobenzyl 2-(2-benzeneseleninylethoxy)-2-vinyl-clavam-3-carboxylate (4.5 g; 8.4 mmol) was heated under reflux in toluene (50 ml) for ½ hour, then cooled and evaporated in vacuo. The resulting oil was subjected to column chromatography using ethyl acetate:toluene (1:7) as eluent. Fractions containing component Rf 0.20 (ethylacetate:toluene; 1:7) were combined and evaporated to give the desired aldehyde as colourless needles, 1.4 g (44% , as a 1:1 mixture of E and Z isomers. νmax ($CHCl_3$) 1810, 1760, 1730 and 1700 $cm^{-1}$. $\delta_H$ ($CDCl_3$). 2.11-2.58 (4H, m, $CH_2CH_2CHO$), 3.06 and 3.19 (1H, 2 x dd, J 16 and 1Hz, 6β-CH), 3.49 and 3.53 (1H, 2 x dd, J4 and 16Hz, 6α-CH), 4.63 and 5.10 (1H, 2 x dt, J8 and 2Hz, $CHCH_2CH_2CHO$), 5.07 and 5.33 (1H, 2 x d, J2Hz, 3-CH), 5.25 and 5.32 (2H, m, $CH_2$- 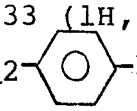 -$NO_2$), 5.65-5.70 (1H, m, 5-CH), 7.45-7.60 and 8.18 to 8.33 (4H, m, ArH), 9.7-9.8 (1H, m, $(CHCH_2)_2CHO$).

Example 33f

p-Nitrobenzyl 2-(4-hydroxybutylidene)clavam-3-carboxylate

p-Nitrobenzyl 2-(4-oxobutylidene)clavam-3-carboxylate (1.2 g, 3.3 mmol) as a 5:3 mixture of Z and E isomers was dissolved in dry dimethoxyethane (20 ml) and cooled to -20°C. Sodium borohydride (0.15 g, 3.96 mmol) was added potionwise over 5 minutes. The reaction was stirred at -20°C for ¼ hour, after which thin layer chromatography showed that all of the starting aldehyde had been consumed, and the reaction was diluted with ethyl acetate (100 ml). The organic phase was washed with saturated brine (2 x 100 ml), dilute hydrochloric acid solution, and sodium bicarbonate then dried over magnesium sulphate. Evaporation and silica gel column chromatography using ethylacetate:toluene (1:1) as eluent gave the Z isomer only. 7.3 mg (6%). $\nu$max ($CHCl_3$) 1800, 1750 and 1700 $cm^{-1}$. $\delta_H$ ($CDCl_3$). 1.35 (1H, broad s, O$\underline{H}$), 1.60 (2H, tt, J7Hz, :$CHCH_2\underline{CH_2}OH$), 2.20 (2H, dt, J7Hz, $CH\underline{CH_2}(CH_2)_2OH$), 3.08(1H, d, J16Hz, 6β-$\underline{CH}$), 3.50(1H, dd, J16 and 3Hz, 6β-$\underline{CH}$), 3.55-3.65(2H, m, $CH(CH_2)_2\underline{CH_2}OH$), 4.63 (1H, dt, J7 and 1Hz, :$\underline{CH}(CH_2)_3OH$), 5.09 (1H, d, J1Hz, 3-$\underline{CH}$), 5.25 and 5.33 (2H, ABq, J12Hz,

$\underline{CH_2}$- 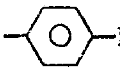 -$NO_2$), 5.68 (1H, d, J3Hz, 5-$\underline{CH}$), 7.52 and 8.25 (4H, ABq, J 8Hz, Ar$\underline{H}$).

Example 34a

Benzyl 2-(4-phenoxybutylidene)clavam-3-carboxylate

To a solution of benzyl 2-(4-hydroxybutylidene)-clavam-3-carboxylate (0.16 g; 0.51 mmol) in tetrahydrofuran (3 ml) containing triphenylphosphine (0.15 g; 0.57 mmol) and phenol (0.07 g; mmol) at 0°C was added diethylazodicarboxylate (0.09 ml; 0.57 mmol). The reaction was stirred at 0°C for ¼ hour then allowed to warm to room temperature. Thin layer chromatography indicated that all of the starting material had been consumed and the reaction mixture was concentrated ( 1 ml) and toluene (20 ml) added. The organic phase was washed with dilute sodium hydroxyide solution (2 x 20 ml), saturated brine (until aqueous washings were neutral) then dried over anhydrous magnesium sulphate. Removal of the solvent *in vacuo* produced a yellow oil which was subjected to silica gel column chromatography using ethyl acetate:cyclohexane (2:3) as eluent. Combining and evaporating fractions containing component Rf=0.54 (ethylacetate:cyclohexane; 2:3) gave the title compound in 46% yield. $\nu$max ($CHCl_3$) 1800, 1750 and 1700 $cm_{-1}$. $\delta_H$ ($CDCl_3$). 1.78(2H, tt, 57.5Hz, $CHCH_2\underline{CH_2}CH_2OPh$), 2.26(2H, dt, J7.5Hz, $CH\underline{CH_2}(CH_2)OPh$), 2.92 (1H, dd, J17 and 1Hz, 6β-C$\underline{H}$); 3.40(1H, dd, H17 and 3Hz, 6α-$\underline{CH}$), 3.88 (2H, t, J7.5Hz, $CH(CH_2)_2\underline{CH_2}OPh$), 4.60 (1H, dt, J7.5 and 2Hz, $C\underline{H}(CH_2)_3OPh$), 5.03 (1H, d, J2Hz, 3-$\underline{CH}$), 5β, 5.22 (2H, ABq, J12.5Hz, $\underline{CH_2}Ar$), 5.58(1H, d, J3Hz, 5-$\underline{CH}$), 6.81-6.98(3H, m, 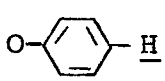 and 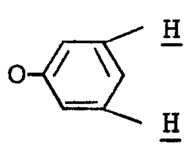), 7.20-7.45 (7H, m, 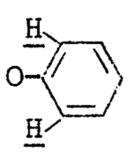 and $CH_2Ar\underline{H}$).

Example 34b

Sodium 2-(4-phenoxybutylidene)-clavam-3-carboxylate

To a solution of benzyl 2-(4-phenoxybutylidene) clavam-3-carboxylate (0.21 g; 0.53 mmol) in tetrahydrofuran (10 ml) palladium on carbon (0.07 g) was added. The mixture was hydrogenated at room temperature under 1 atmosphere of hydrogen for ½ hour then filtered. The filtrate was reduced to a small volume ( 1ml) then partitioned between ethylacetate and water. The pH was adjusted to 7.5 with sodium hydroxide. Separation of the aqueous layer and evaporation produced a pale yellow glass which gave the title compound as an off white solid in 81% yield, after trituration with acetonitrile. $\nu$max (KBr) 1780, 1700 and 1610cm$^{-1}$. $\delta_H$ ($D_2O$) 1.75-1.95 (2H, m, $CHCH_2CH_2CH_2OPh$), 2.13-2.43 (2H, m, $CHCH_2(CH_2)_2OPh$) 2.73 (1H, d, J 17Hz, 6β-CH), 3.40 (1H, dd, J17 and 3Hz, 6α-CH), 4.06 (2H, t, J7Hz, $:CH(CH_2)_2CH_2OPh$), 4.72 (2H, t, J7Hz, $:CH(CH_2)_3OPh$), 4.85 (1H, d, J1.5Hz, 3-CH), 5.51 (1H, d, J3Hz, 5-CH), 6.95-7.10 (3H, m, 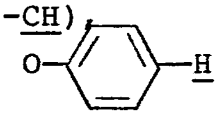

and 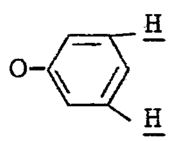 ), 7.30-7.45 )2H, m, 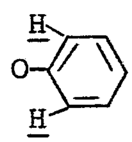 ).

Example 35a

Benzyl 2-(4-N-benzyloxycarbonyl-p-toluenesulphonamido-butylidene)clavam-3-carboxylate

Benzyl 2-(4-hydroxybutylidene)clavam-3-carboxylate (0.4 g, 1.266 mmol) eas dissolved in dry redistilled tetrahydrofuran (8 ml) containing triphenylphosphine (0.365 g, 1.39 mmol) and (N-benzyloxycarbonyl)-p-toluenesulphonamide (0.6 g, 1.97 mmol). This mixture was placed in a dry-ice/carbontetrachloride cooling bath and was immediately treated with a tetrahydrofuran (dry and redistilled, 5 ml) solution of diethylazodicarboxylate (0.22 g, 1.2632 mmol) with vigorous stirring. After 5 minutes the reaction was allowed to warm to room temperature and after a further 15 minutes was diluted with ethyl acetate, washed with brine, dried ($MgSO_4$) and evaporated. The residue was chromatographed on silica-gel (toluene/ethylacetate, 7:1 as eluent) affording the title compound, 0.5105 g (67% yield). $\nu$max (film) 1802, 1740 sh, 1731, and 1701 $cm^{-1}$. $\delta$($CDCl_3$) 1.6-2.25 (4H, m, $\underline{CH_2}$,$\underline{CH_2}$N), 2.33 and 2.36 (3H, each s, rotomeric C$\underline{H}_3$), 2.94 (1H, d, J17Hz, 6β-$\underline{CH}$), 3.46 (1H, d, J17 and 3Hz, 6α-C$\underline{H}$), 3.63-3.96 (2H, m, C:C.C$\underline{H}_2$), 4.56 (1H, brt, J7Hz C:C$\underline{H}$), 5.02 (1H, brs, 3-C$\underline{H}$), 5.07 and 5.18 (4H, each s, $CO_2$C$\underline{H}_2$x2), 5.62 (1H, d, J3Hz, 5-C$\underline{H}$) and 7.00-7.800 (14H, m, aromatics).

Example 35b

Sodium 2-(4-p-toluenesulphonamido-butylidene)clavam-3-carboxylate

Benzyl 2-(4-[N-benzyloxycarbonyl]p-toluene sulphonamidosulphonamido)butylidene-clavam-3-carboxylate (0.5105g, 0.845 mmol) in aqueous (1 ml) tetrahydrofuran (12 ml) containing sodium hydrogen carbonate (0.071 g, 0.845 mmol) and a catalyst (Pd/C, 10% 0.1 g) was hydrogenated for 1 hour. At the end of this time the reaction mixture was filtered and the filtrate evaporated. The residue was taken up into dry acetone and filtered and the title compound was obtained as a white solid upon concentration of the solvent, 0.0155 g (4.5% yield). $\nu$max (Nujol) 3270, 1789, 1701 and 1600 $cm^{-1}$. $\delta$($D_2O$) 1.38-1.57 (2H, m, $CH_2$.$C\underline{H}_2$.$CH_2$), 1.91-2.13 (2H, m, $C\underline{H}_2N$), 2.42 (3H, s, $C\underline{H}_3$), 2.79-2.92 (2H, m, C:C.$C\underline{H}_2$), 2.98 (1H, d, J17Hz 6β-C$\underline{H}$), 3.52 (1H, dd, J17 and 2Hz 6α-C$\underline{H}$), 4.53 (1H, br t, J8Hz C:C$\underline{H}$), 4.80 (2H, s, $C\underline{H}_2Ph$), 4.82 (1H, s, 3-C$\underline{H}$), 5.60 (1H, d, J 2Hz, 5-C$\underline{H}$) and 7.45, 7.73 (4H, AA'XX', J13 Hz, Ar$\underline{H}$).

Example 36a

Benzyl 2-(4-acetoxybutylidene)clavam-3-carboxylate

A solution of benzyl 2-(4-hydroxybutylidene)clavam-3-carboxylate (0.19 g; 0.6 mmol) in dichloromethane (2 ml) was treated with pyridine (0.06 ml; 0.7 mmol) and acetyl chloride (0.05 ml; 0.7 mmol) at 0°C for 5 min, after which thin layer chromatography indicated that all of the starting alochol had been consumed. The solution was diluted to 25 ml with dichloromethane and the organic phase washed with water (2 x 25 ml), dilute hydrochloric acid (1 x 25 ml), saturated sodium bicarbonate solution (1 x 25 ml), saturated brine (2 x 25 ml) and dried over anhydrous magnesium sulphate. Removal of the solvent under reduced pressure gave the title compound as a colourless oil (yield 88%).

$\nu_{max.}$ (5% $CHCl_3$) 1800, 1735 and 1700 $cm^{-1}$. $\delta_H$ ($CDCl_3$) 1.64 (2H, tt, J 7 Hz, $CH_2CH_2\underline{CH_2}CH_2O-$) 2.04 (3H, s, $\underline{CH_3}CO$) 2.14 (2H, dt, J 7 Hz, $CH\underline{CH_2}(CH_2)_2O-$), 3.05 (1H, d, J 17 Hz, 6β-$\underline{CH}$), 3.46 (1H, dd, J 17 and 3 Hz, 6α-$C\underline{H}$), 4.00 (2H, t, J 7 Hz, $CH(CH_2)_2\underline{CH_2}O-$) 4.56 (1H, dt, J 7 and 1 Hz, $\underline{CH}(CH_2)_3O$) 5.03 (1H, d, J 1 Hz, 3-$\underline{CH}$), 5.15 and 5.24 (2H, ABq, J 12 Hz, $\underline{CH_2}Ph$), 5.65 (1H, d, J 3 Hz, 5-$\underline{CH}$), 7.30 - 7.45 (5H, m, $Ar\underline{H}$).

Example 36b

Lithium 2-(4-acetoxybutylidene)clavam-3-carboxylate

Palladium on barium sulphate (0.08 g) was prehydrogenated at room temperature in tetrahydrofuran (5 ml) for 5 min. then benzyl 2-(4-acetoxybutylidene)-clavam-3-carboxylate (0.21 g; 0.58 mmol) in tetrahydrofuran (10 ml) was added. The reaction mixture was hydrogenated for 0.75 hr, after which thin layer chromatography indicated that all of the starting ester had been consumed and the catalyst was filtered off. The catalyst was washed with tetrahydrofuran (20 ml) and the combined filtrates reduced to a small volume (≃ 1 ml) then partitioned between ethyl acetate and water. Adjustment of the pH to 7.2 with lithium hydroxide followed by separation of the aqueous layer and removal of the water, under reduced pressure, gave an off-white solid, which was washed with tetrahydrofuran and filtered, then dried in vacuo to give the title compound (yield 61%).

$\nu_{max.}$ (KBr) 1780, 1700 (sh) and 1615 $cm^{-1}$. $\delta_H$ ($D_2O$) 1.74 (2H, tt, J 6.8 Hz, $CHCH_2\underline{CH_2}CH_2O$), 2.07 (3H, s, $\underline{CH_3}O$), 2.10 - 2.28 (2H, m, $CH\underline{CH_2}(CH_2)_2O$), 3.05 (1H, d, J 18 Hz, 6β-$\underline{CH}$), 3.54 (1H, dd, J 18 and 2.7 Hz, 6α-$\underline{CH}$) 4.08 (2H, t, J 6.8 Hz, $CH(CH_2)_2\underline{CH_2}O$), 4.70 (1H, dt, J 6.8 and 1 Hz, $\underline{CH}(CH_2)_3O$), 4.87 (1H, d, J 1 Hz, 3-$\underline{CH}$), 5.65 (1H, d, J 2.7 Hz, 5-$\underline{CH}$),

Example 37a

Benzyl 2-(4-benzoyloxybutylidene)clavam-3-carboxylate

To a solution of benzyl 2-(4-hydroxybutylidene)-clavam-3-carboxylate (0.2 g; 0.63 mmol) in dichloromethane (2 ml) at 0°C was added pyridine (0.06 ml; 0.7 mmol) and benzoyl chloride (0.08 ml; 0.7 mmol). The reaction mixture was stirred at 0°C for 5 min. after which thin layer chromatography indicated that all of the starting alcohol had been consumed. The solution was diluted with dichloromethane (20 ml) and the organic phase washed with water (2 x 25 ml), dilute hydrochloric acid (1 x 25 ml), saturated sodium bicarbonate solution (1 x 25 ml), saturated brine (2 x 25 ml) and dried over anhydrous magnesium sulphate. Removal of the solvent gave a yellow oil which was subjected to silica gel column chromatography eluting with ethyl acetate : cyclohexane (2 : 3). Fractions were combined which contained the component Rf = 0.56 (ethyl acetate : cyclohexane; 2 : 3) and evaporated *in vacuo* to give the title compound as a colourless oil (yield 81%).

$\nu_{max.}$ (5% $CHCl_3$) 1800, 1745 and 1710 $cm^{-1}$. $\delta_H$ ($CDCl_3$) 1.78 (2H, tt, J 7 Hz, $CHCH_2\underline{CH_2}CH_2O-$), 2.24 (2H, dt, J 7 Hz, $CH\underline{CH_2}(CH_2)_2O-$), 3.02 (1H, dd, J 17 and 1 Hz, 6β-$\underline{CH}$), 3.42 (1H, dd, J 17 and 3 Hz, 6α-$\underline{CH}$), 4.28 (2H, t, J 7 Hz, $CH(CH_2)_2\underline{CH_2}O-$), 4.60 (1H, dt, J 7 and 1 Hz, $\underline{CH}(CH_2)_3O-$), 5.04 (1H, d, J 1 Hz, 3-$\underline{CH}$), 5.16 and 5.25 (2H, ABq, J 12.5 Hz, $\underline{CH_2}Ph$), 5.62 (1H, d, J 3 Hz, 5-$\underline{CH}$), 7.30 - 7.38 (5H, m, $CH_2\underline{Ph}$), 7.39 - 7.50 (2H, m, 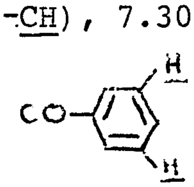 ), 7.51 - 7.62 (1H, m, 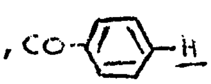 ), 8.00 - 8.12 (2H, m, 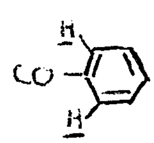 ).

Example 37b

Lithium 2-(4-benzoyloxybutylidene)clavam-3-carboxylate

Palladium on barium sulphate (0.07 g) was prehydrogenated at room temperature in tetrahydrofuran (5 ml) for 5 min., then a solution of benzyl 2-(4-benzoyloxybutylidene)clavam-3-carboxylate (0.20 g; 0.47 mmol) in tetrahydrofuran (10 ml) was added. The reaction mixture was hydrogenated for 1 hr, after which thin layer chromatography indicated that all of the starting ester had been consumed and the catalyst was filtered off. The catalyst was washed with tetrahydrofuran (20 ml) and the combined filtrates reduced to a small volume and partitioned between ethyl acetate and water. Adjustment of the pH to 7.2 with lithium hydroxide followed by separation of the aqueous layer and removal of the water under reduced pressure gave an off-white solid which was washed with tetrahydrofuran and filtered, then dried *in vacuo* to give the title compound (yield 68%).

$\nu_{max.}$ (KBr) 1785, 1715, 1700 (sh) and 1610 $cm^{-1}$.
$\delta_H$ ($D_2O$) 1.83 - 2.01 (2H, m, $CHCH_2\underline{CH_2}CH_2O$-), 2.16 - 2.44 (2H, m, $CH_2\underline{CH_2}(CH_2)_2O$-), 2.84 (1H, d, J 16 Hz, 6β-CH)
3.42 (1H, dd, J 16 and 3 Hz, 6α-CH)
4.34 (2H, t, J 6.7 Hz, $CH(CH_2)_2\underline{CH_2}O$-), 4.76 (1H, t, J 6.7 Hz, $\underline{CH}(CH_2)_3O$-, partly obscured by HOD peak), 5.58 (1H, d, J 3 Hz, 5-CH), 7.55 (2H, dd, J 8.3 Hz,
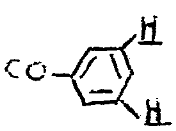 ), 7.70 (1H, dd, J 8.3 Hz, 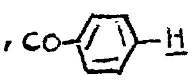 )
8.05 (2H, d, J 8.3 Hz, 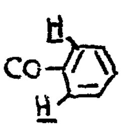 ).

Example 38a

Benzyl 2-[4-(p-toluenesulphonamidocarbonyloxybutylidene] clavam-3-carboxylate

Benzyl 2-(4-hydroxybutylidene)clavam-3-carboxylate (227 mg; 0.716 mmol) in dichloromethane (5 $cm^3$) at -10° was treated with 1 equivalent of p-toluenesulphonyl-isocyanate and stirred for ½ hour allowing the temperature to rise to 10°. The solvent was evaporated and the residue chromatographed on silica eluting with ethyl acetate-cyclohexane (1:1). Fractions were collected containing the title compound Rf ($SiO_2$/ethyl acetate-cyclohexane (1:1)) = 0.43 (detection by aqueous potassium permanganate spray). Combined fractions were evaporated to afford 380 mg of an oil. ν (film) 1800, 1750, 1700 (shoulder), the proton n.m.r. spectra was consistent with the desired product.

Example 38b

Sodium 2-[4-(p-toluenesulphonamidocarbonyloxybutylidene] clavam-3-carboxylate

Benzyl 2-[4-(p-toluenesulphonamidocarbonyloxybutylidene]-clavam-3-carboxylate (200 mg; 0.389 mmol) in tetrahydrof an (10 $cm^3$) was hydrogenolysed at atmospheric pressure in the presence of 10% palladium on charcoal (70 mg) for 30 minutes. The catalyst was filtered off and the filtrate diluted to 25 cm3 with tetrahydrofuran and 25 $cm^3$ water. The pH was adjusted to 7.2 using dilute sodium hydroxide. The tetrahydrofuran was evaporated under vacuum and the aqueous residue diluted with water and washed several times with ethyl acetate. The aqueous phase was evaporated and triturated with tetrahydrofuran/acetonitrile to form a pale buff coloured solid, yield = 110 mg ν (Nujol) 1785, 1700, 1620, 1280, 1145, 1120, 1072, 895, 860, 810, 790 $cm^{-1}$. The proton n.m.r. was consistent with the desired compound

0080286

Example 39a

Benzyl 2-[4-(3-methyl-2,4,5-trioxoimidazolidinyl)-butylidene]clavam-3-carboxylate

Benzyl 2-(4-hydroxybutylidene)clavam-3-carboxylate (0.388 g; 1.224 mmol) in dry redistilled tetrahydrofuran (5 ml) containing triphenylphosphine (0.353 g, 1.346 mmol) and N-methyl-2,4,5-trioxoimidazolidine (0.235 g, 1.836 mmol) was placed under a stream of dry nitrogen at 0°. Neat diethylazodicarboxylate (0.234 g, 1.346 mmol) was then added and after 20 minutes the reaction was diluted with ethylacetate and washed with brine (2 x). The organic layer was dried and evaporated prior to silica-gel chromatography (toluene/ethylacetate 7:1). The title ester was thus obtained in 93% yield (0.488 g).

$\nu_{max.}$ (film) 1801, 1779, 1732 and 1703 sh $cm^{-1}$ δ ($CDCl_3$) 1.50 - 2.23 (4H, m, :C.$C\underline{H}_2$.$C\underline{H}_2$), 2.72 (1H, d, J 16 Hz, 6β-C$\underline{H}$), 3.23 (3H, s, $C\underline{H}_3$), 3.26 - 3.76 (3H, m, 6α-C$\underline{H}$ and $C\underline{H}_2$.N), 4.55 (1H, br. t, J 6 Hz, 8-C$\underline{H}$), 5.03 (1H, br. s, 3-C$\underline{H}$), 5.19 (2H, s, $OC\underline{H}_2C_6H_6$), 5.63 (1H, d, J 3 Hz, 5-C$\underline{H}$) and 7.36 (5H, s, Ar$\underline{H}$).

0080286

Example 39b

Sodium 2-[4-(3-methyl-2,4,5-trioxoimidazolidinyl)-butylidene]clavam-3-carboxylate

To a solution of benzyl 2-[4-(3-methyl-2,4,5-trioxoimidazolidinyl)butylidene]clavam-3-carboxylate (0.1 g; 0.23 mmol) in tetrahydrofuran (5 ml) was added 10% palladium on carbon (0.02 g). The mixture was hydrogenated at room temperature for a quarter of an hour, after which thin layer chromatography indicated that all of the starting ester had been consumed. The catalyst was filtered off, washed with tetrahydrofuran (15 ml) and the combined filtrates were reduced to a small volume and partitioned between ethyl acetate (10 ml) and water (15 ml). The pH was adjusted to 7.5 with sodium hydroxide solution and the aqueous layer separated. Removal of the water produced a colourless glass which gave the title compound in 75% yield as a white solid after trituration with acetone.

$\nu_{max.}$ (Nujol) 1790, 1740, 1700 and 1610 $cm^{-1}$.

Example 40

Benzyl 2-(4-trifluoromethanesulphonyloxybutylidene)-clavam-3-carboxylate

Benzyl 2-(4-hydroxybutylidene)clavam-3-carboxylate (0.448 g, 1.4132 mmol) in dry toluene (8 ml) was placed under an atmosphere of dry nitrogen and cooled to -20°C. Triethylamine (0.197 ml, 1.413 mmol) was then added followed by trifluoromethanesulphonic anhydride (0.23775 ml, 1.413 mmol) in dry toluene (3 ml). The reaction was judged to be complete after 1 h (t.l.c.) and was diluted with ethylacetate and washed with brine (2 x) and saturated sodium hydrogencarbonate solution. The organic layer was dried ($MgSO_4$) and evaporated to give the title ester in 91% yield (0.5782 g) which may be used without further purification. $\nu_{max.}$ (film) 1803, 1754 and 1702 $cm^{-1}$. δ ($CDCl_3$) 1.40 - 2.50 (4H, m, :C.$C\underline{H}_2$.$C\underline{H}_2$), 3.02 (1H, d, J 18 Hz, 6β-C$\underline{H}$), 3.50 (1H, dd, J 18 and 3 Hz, 6α-C$\underline{H}$), 4.24 - 4.66 (3H, m, 8-C$\underline{H}$ and $C\underline{H}_2$.O.S), 5.07 (1H, br. s, 3-C$\underline{H}$), 5.20 (2H, s, $CO_2C\underline{H}_2$), 5.67 (1H, d, J 3 Hz, 5-C$\underline{H}$), and 7.38 (5H, s, $C_6\underline{H}_6$).

Example 41

Benzyl 2-(4-phenylthiobutylidene)clavam-3-carboxylate

A solution of thiophenol (.04 ml) in dimethylformamide (4 ml) was cooled in an ice-bath and sodium hydride (16 mg of 50% mineral oil dispersion) was added. When the solution became clear a solution of benzyl 2-[4-(p-toluenesulphonyloxy)butylidene ]clavam-3-carboxylate (139 mgs) in dimethylformamide (2 ml) was added. The mixture was stirred for 1 hr and allowed to warm to room temperature. Acetic acid (0.1 ml) was then added and the solution was diluted with ethyl acetate. The ethyl acetate solution was washed successively with sodium bicarbonate solution, three portions of water, and brine. The solution was dried over magnesium sulphate and evaporated, the product was obtained by column chromatography of the residue (Kieselgel 4 : 1 petroleum ether : ethyl acetate as eluent. Yield, 53 mgs.

I.r. $\nu_{max.}$ ($CHCl_3$) 1803, 1750 and 1698 $cm^{-1}$.

N.m..r. $\delta$ ($CDCl_3$) 1.55 (2H, quintet, J 7 Hz), 2.14 (2H, q, J 7 Hz) 2.77 (2H, t, J 7 Hz), 2.90 (1H, d, J 17 Hz), 3.38 (1H, dd, J 3 and 17 Hz), 4.47 (1H, broad t, J 7 Hz), 4.93 (1H, s), 5.08 (2H, s), 5.53 (1H, d, J 3 Hz) 7.1 - 7.3 (10H, m).

0080286

Example 42

Benzyl E-2-{4-(p-toluenesulphonyloxy)butylidene}-clavam-3-carboxylate

Benzyl E-2-(4-hydroxybutylidene)clavam-3-carboxylate (240 mg; 0.76 mmol) in dry dichloromethane (3 ml) was treated sequentially with p-toluene sulphonyl chloride (289 mg; 1.5 mmol) and dry pyridine (183 ml; 2.27 mmol). The reaction mixture was stirred at room temperature for 24 hours. After which t.l.c. (EtOAc:cyclohexane, Rf=0.67) showed the reaction to be complete. Solvent was removed in vacuo and the residue taken up in ethyl acetate and washed with 20% citric acid solution, water and brine; the organic phase was separated, dried ($MgSO_4$) and evaporated. The residual oil was fractionated on silica gel, elution with ethyl acetate:cylohexane 1:2 gave the required tosylate in 65% yield. $\nu_{max}$ (film) 1800, 1750, 1700. $\delta$ ($CDCl_3$) 1.53 (2H, m, $CH_2C\underline{H}_2CH_2OSO_2$), 1.97 (2H, m, $C\underline{H}_2CH_2CH_2OSO_2$), 2.45 (3H, s, $C\underline{H}_3$), 3.0 (1H, d, J17Hz, 6β-C$\underline{H}$) 3.43 (1H, dd, J17 and 3Hz, 6α-C$\underline{H}$), 3.9 (2H, t, J6.5Hz, $CH_2CH_2C\underline{H}_2OSO_2$), 4.91 (1H, dt, J7 and 2Hz, 8-C$\underline{H}$), 5.12 (1H, bs, 3-CH), 5.17 (2H, s, $CO_2C\underline{H}_2$), 5.63 (1H, d, J3Hz, 5α-C$\underline{H}$), 7.36 (7H, m, $C_6\underline{H}_5$ and 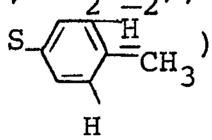), 7.7 (2H, $d_{AB}$, J 8Hz, 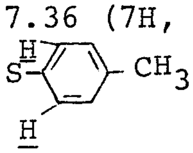).

Example 43

Conversion of benzyl E-2-(4-hydroxybutylidene)clavam-3-carboxylate to benzyl Z-2-(4-hydroxybutylidene)clavam-3-carboxylate

A solution of benzyl E-2-(4-hydroxybutylidene)clavam-3-carboxylate (50 mg) in dry benzene (100 ml) was irradiated under nitrogen in a quartz vessel using a Hanovia low pressure lamp with maximum at 254 n.m. n.m.r. of the mixture after 6 hours showed a 60/40 mixture of Z/E isomers.

Example 44

Benzyl Z-2-(4-iodobutylidene)clavam-3-carboxylate

Benzyl Z-2-(4-hydroxybutylidene)clavam-3-carboxylate (134 mg; 0.42 mmol) in dry dimethylformamide (8 ml) was treated with triphenyl phosphite methiodide (229 mg; 0.51 mmol), at room temperature, under a nitrogen atmosphere. The reaction was stirred at room temperature overnight; solvent was removed *in vacuo* and the residual oil was dissolved in ethyl acetate, washed with water, brine and dried ($MgSO_4$). Evaporation of the solvent gave a yellow oil which was fractionated on silica gel, (n.m.r. of the crude material before chromatography was consistent with the correct structure).

Example 45

Benzyl 2-{4-(N-acetyl-N-benzyloxycarbonyl)aminobutylidene}-clavam-3-carboxylate

To a solution of benzyl 2-(4-hydroxybutylidene)clavam-3-carboxylate (0.388g; 1.22 mmol) in tetrahydrofuran (5 ml) containing triphenylphosphine (0.35 g; 1.33 mmol), and N-benzyloxycarbonylacetamide (0.35 g; 1.82 mmol) at $0^oC$ was added diethylazodicarboxylate (0.21 ml; 1.33 mmol). The reaction mixtures was stirred at $0^oC$ for ½ hour then allowed to warm to room temperature. Thin layer chromatography indicated that all of the starting material had been consumed and the reaction mixture was reduced to small volume ( 1ml) and toluene (30 ml) added. The organic phase was washed with saturated brine (2 x 30 ml) and dried over anhydrous magnesium sulphate. Removal of the solvent gave a yellow oil which was subjected to silica gel column chromatography using ethyl acetate: cyclohexane (2:3) as elutent. Fractions containing the component Rf=0.50 (ethyl acetate;cyclohexane; 2.3) were combined and evaporated to give the title compound in 28% yield as a colourless oil.

$\delta_H$ ($CDCl_3$) 1.00-2.26 (4H, m, $CH\underline{CH_2}(CH_2)_2N$ and $CHCH_2\underline{CH_2}CH_2N$), 2.45 (3H, s, $N\underset{\overset{\|}{O}}{C}\underline{CH_3}$), 2.92 (1H, d, J 17Hz, 6β-$\underline{CH}$), 3.42 (1H, dd, J 17 and 2Hz, 6α-$\underline{CH}$, partially obscured by t at δ 3.68), 3.68 (2H, broad t, J 7Hz, $CH(CH_2)_2CH_2N$, partially obscured by dd at δ 3.42), 4.50 (1H, broad, J Hz, $\underline{CH}(CH_2)_3N$), 4.94 (1H, broad s, 3-$\underline{CH}$), 5.16 and 5.20 (4H, 2 x s, $NCO_2\underline{CH_2}Ph$ and $C.CO_2\underline{CH_2}Ph$), 5.55 (1H, d, J 2Hz, 5-$\underline{CH}$), 7.25 and 7.30 (10H, 2 x s, Ar$\underline{H}$).

CLAIMS

1. A 3-carboxyclavam derivative characterised in that it comprises a butylidene moiety at the 2-position of the clavam nucleus.

2. A compound as claimed in claim 1 having the formula (III):

(III) 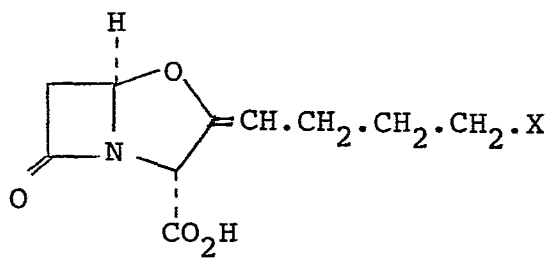

or a salt or ester thereof wherein X is hydrogen, hydroxy, substituted hydroxy, mercapto, substituted mercapto, azido, cyano, halo, nitro, amino, substituted amino, isothiocyanato, or X represents the residue of a carbon nucleophile or is an activated aryl or heteroaryl group.

3. A compound as claimed in claim 2 having the Z sterochemistry about the double bond.

4. A compound as claimed in either of claims 2 or 3 in the form of the free acid or pharmaceutically acceptable salt or ester thereof.

5. A compound as claimed in any one of claims 2 to 4 wherein X is hydroxy, $C_{1-10}$ alkoxy, $C_{1-10}$ alkanoyloxy, benzoyloxy, $C_{1-10}$ alkylthio, benzoylthio, azido, halo, nitro, amino, $C_{1-10}$ alkylamino, $C_{1-10}$ alkanoylamino, arylsulphonylamino or heterocyclic.

6. A compound as claimed in any one of claims 2 to 5 wherein X is hydroxy.

7. A process for the preparation of a compound as claimed in claim 2 which process comprises the reduction of a compound of formula (V):

(V)

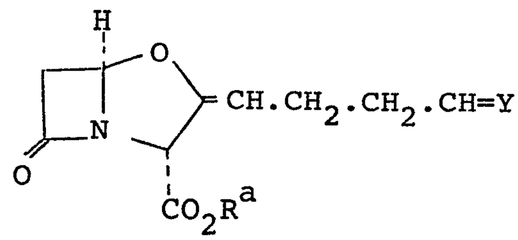

where $R^a$ is hydrogen or a carboxy-blocking group and Y is an oxygen or sulphur atom, and thereafter if necessary:

i) converting the product of formula (III) wherein X is hydroxy or mercapto to a compound of formula (III) wherein X is other than hydroxy or mercapto;

ii) removing any carboxy-blocking group $R^a$;

iii) converting the product into a salt or ester.

8. A pharmaceutical composition which comprises a compound as claimed in any one of claims 1 to 6 in the form of the free acid or pharmaceutically acceptable salt or ester, together with a pharmaceutical acceptable carrier.

9. A composition as claimed in claim 8 which further comprises a antibacterially active β-lactam compound.

10. A composition as claimed in claim 9 wherein the β-lactam is a penicillin or cephalosporin.

0080286

European Patent Office

# EUROPEAN SEARCH REPORT

Application number

EP 82 30 5886

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | GB-A-1 546 569 (BEECHAM) *Claims* | 1-4,7-10 | C 07 D 498/04<br>A 61 K 31/42<br>(C 07 D 498/04, 263/00,205/00)<br>(A 61 K 31/42, 31/43)<br>(A 61 K 31/42, 31/545) |
| Y | GB-A-1 593 275 (GLAXO) | 1-4,7-10 | |
| Y | EP-A-0 002 319 (BEECHAM) *Claims* | 1-4,7-10 | |
| Y | EP-A-0 018 305 (HOECHST) *Claims 30-59* | 1,8 | |
| A | US-A-4 293 555 (B.G.CHRISTENSEN) *Column 1, lines 16-45* | 1,8 | TECHNICAL FIELDS SEARCHED (Int. Cl. 3)<br>C 07 D 498/00<br>A 61 K 31/00 |

The present search report has been drawn up for all claims

| Place of search | Date of completion of the search | Examiner |
|---|---|---|
| THE HAGUE | 14-02-1983 | CHOULY J. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document